# EUROPEAN PATENT SPECIFICATION

(11) **EP 4 331 579 B1**
(45) Date of publication and mention of the grant of the patent: **11.11.2026**
(21) Application number: 22796090.3
(22) Date of filing: 26.04.2022
(51) Int. Cl.: A61K 31/4015, A61P 21/04, A23L 33/10, A61P 21/00

(54) **COMPOSITION COMPRISING OXIRACETAM FOR PREVENTING OR TREATING MUSCULAR DISEASE**
ZUSAMMENSETZUNG MIT OXIRACETAM ZUR PRÄVENTION ODER BEHANDLUNG VON MUSKELERKRANKUNGEN
COMPOSITION COMPRENANT DE L'OXIRACÉTAM POUR LA PRÉVENTION OU LE TRAITEMENT D'UNE MALADIE MUSCULAIRE

(30) Priority: 26.04.2021 KR 20210053617; 01.07.2021 KR 20210086607; 29.10.2021 KR 20210146501
(43) Date of publication of application: 06.03.2024
(73) Proprietor: KSB Tugen Inc., Seoul 05029 (KR)
(72) Inventor: KIM, Bokyung, Seoul 04978 (KR); JUNG, Seung Hyo, Seoul 02252 (KR); LEE, Kyung-Jin, Seoul 05021 (KR); KIM, Su Jung, Anseong-si Gyeonggi-do 17590 (KR)
(74) Representative: Schiweck Weinzierl Koch Patentanwälte Partnerschaft mbB
(86) International application number: PCT/KR2022/005916
(87) International publication number: WO 2022/231257

(56) References cited:
- WO-A1-2014/181101
- WO-A1-2017/043935
- WO-A1-2020/101042
- KR-A- 20000 009 465
- KR-A- 20030 042 883
- KR-A- 20100 126 454
- KR-A- 20190 053 253
- MONDADORI C ET AL: "SYNERGISTIC EFFECTS OF OXIRACETAM AND PIRACETAM IN COMBINATION WITH ANTIEPILEPTIC DRUGS", ACTA NEUROLOGICA SCANDINAVICA, MUNKSGAARD, COPENHAGEN, DK, vol. 74, no. SUPPL. 109, 1 January 1986 (1986-01-01), pages 113 - 116, XP009035048, ISSN: 0001-6314
- HALL E D ET AL: "Facilitatory effects of piracetam on excitability of motor nerve terminals and neuromuscular transmission", NEUROPHARMACOLOGY, ELSEVIER, AMSTERDAM, NL, vol. 26, no. 11, 1 November 1987 (1987-11-01), pages 1573 - 1579, XP023754378, ISSN: 0028-3908, [retrieved on 19871101], DOI: 10.1016/0028-3908(87)90003-7
- SUN YUZHEN, XU BAOQUAN, ZHANG QIANG: "Nerve growth factor in combination with Oxiracetam in the treatment of Hypertensive Cerebral Hemorrhage", PAKISTAN JOURNAL OF MEDICAL SCIENCES, vol. 34, no. 1, 1 January 2018 (2018-01-01), pages 73 - 77, XP055982396, DOI: 10.12669/pjms.341.13395

## Description

### [Technical Field]

The present invention relates to a pharmaceutical composition for use in preventing or treating a muscle disease, which comprises oxiracetam or a pharmaceutically acceptable salt or hydrate thereof as an active ingredient, and wherein the muscle disease is any one or more selected from the group consisting of atony, muscular atrophy, muscular dystrophy, muscular degeneration, myasthenia, myositis, cachexia, and senile sarcopenia.

### [Background Art]

Skeletal muscles can be divided into active muscles and postural muscles, and when muscle mass is reduced, muscular atrophy in which the volume of muscle fibers decreases occurs.

Skeletal muscular atrophy or sarcopenia can generally occur naturally as aging progresses, and may secondarily occur due to the non-use of muscles or lack of exercise, or other pathological conditions (cancer, renal failure, lung disease, sepsis, starvation, anticancer treatment, inflammation, stress hormones, etc.). Such muscular atrophy becomes a vicious cycle that causes problems such as weakness, activity impairment and a long-term recovery period, based on the weakening of grip strength for physical activity. Therefore, it is one of the big problems that must be solved in order to pursue a healthy and decent human life away from accelerating aging and the increase in diseases around the world.

Various studies are being conducted on how to effectively control sarcopenia. For example, a growth hormone (GH) has been developed to increase muscle mass, but it is very expensive and has problems of causing some undesirable side effects, such as a shortening of life expectancy. Mitochondrial stabilization drugs also had difficulty in penetrating the therapeutic drug into the mitochondria and affect parts other than the mitochondria, so they did not have an effect on muscular atrophy due to the occurrence of an off-target effect. In addition, although the development of drugs and technology for treatment of sarcopenia capable of inducing muscle regeneration and differentiation is a major task, it is a very inappropriate method for patients or patients lying on a bed. Therefore, additional research and drug development for sarcopenia including senile sarcopenia and muscular atrophy are required.

Muscular dystrophy is a hereditary muscle disease caused by genetic mutations, which is different from sarcopenia caused by aging, and muscular atrophy caused by the decline in activity, denervation, nutritional deficiency, and steroids.

Among types of muscular dystrophy, those caused by mutations in the dystrophin gene on the X chromosome include Duchenne muscular dystrophy (DMD) and mild-type Becker muscular dystrophy (BMD). DMD is a disease with the highest frequency among progressive types of muscular dystrophy, and is known to generally occur in boys aged 2 to 5 years.

Dystrophin is a large gene with a full length of 2.2 Mbp (base pairs) and present between chromosomes Xp21.2 and Xp21.1, but the region that is translated into a dystrophin protein is only 11 kbp. Muscular dystrophin is present right below the muscle membrane and is a key protein of the dystrophin-associated glycoprotein complex connecting the cytoskeleton and the extracellular matrix, involved in the damage to the muscle cell membrane due to muscle contraction.

DMD progresses very rapidly, 90% or more of patients lose their walking function before the age of 12, and after the age of 18, heart failure and electrocardiogram abnormalities caused by myocardial dysfunction are found. Most patients die before the age of 30 due to respiratory disorders caused by trunk muscle atrophy, and spinal and thoracic deformities leading to death before their thirties.

To date, there are few effective drugs for muscular dystrophy. Steroid administration for muscular dystrophy has the effect of prolonging the walking period, but it cannot stop or reverse the progression, and there is a problem in that side effects caused by long-term administration must be tolerated. In addition, gene therapy has been developed, but there is a limitation in that only about 10 to 13% of patients can use exon skipping gene therapy. In addition, there are problems in that subcutaneous or intravenous administration is required every week, and the treatment of myocardial diseases cannot be expected because the penetration into cardiomyocytes is not smooth muscle, and there are very few patients that can also use nonsense mutation therapy (approximately 11 to 14%). Gene therapy using viruses also has a problem in that gene delivery is impossible since the size of the dystrophin gene (cDNA length: approximately 14 kb) exceeds the capacity in a vector. To overcome this, micro-dystrophin in which only 4 rod repeating structures among the 24 structures in the middle of the dystrophin remain has been developed, but there is a problem that side effects of the immune response caused by an AAV vector appear or the expression of dystrophin is very limited.

Therefore, there is an urgent need to develop therapies that are more fundamental and have no side effects to treat muscle diseases including sarcopenia and muscular dystrophy.

[Related Art Documents] Sun Y., Xu B., and Zhang Q., Nerve growth factor in combination with Oxiracetam in the treatment of Hypertensive Cerebral Hemorrhage, Pak J Med Sci. 2018; 34(1):73-77, describes that the nerve growth factor (NGF) in combination with oxiracetam is effective in treating hypertensive cerebral hemorrhage.

[Patent Documents] WO 2017/043935 A1 describes a composition for promoting the differentiation of myoblasts comprising sobrerol or a pharmaceutically acceptable salt thereof, a pharmaceutical composition for preventing or treating a disease associated with weakness in the muscle. WO 2020/101042 A1 discloses polynucleotide, comprising the following base sequences: (a) a base sequence encoding a fusion protein of a nuclease-deficient CRISPR effector protein and a transcription activator, and (b) a base sequence encoding a guide RNA targeting a continuous region of 18 to 24 nucleotides in length in a region set forth in certain sequences in the expression regulatory region of human Utrophin gene.

Korean Unexamined Patent Application Publication No. 10-2021-0093954

Korean Unexamined Patent Application Publication No. 10-2018-0010961

### [Disclosure]

### [Technical Problem]

As a result of examining materials that have an excellent effect of preventing or treating a muscle disease and are safely applied, the present inventors confirmed that oxiracetam, which is a nootropic of the racetam family, has an activity of preventing or treating a muscle disease, and the present invention was completed.

Therefore, the present invention is directed to providing a pharmaceutical composition for use in preventing or treating a muscle disease, which comprises oxiracetam or a pharmaceutically acceptable salt or hydrate thereof as an active ingredient, and wherein the muscle disease is any one or more selected from the group consisting of atony, muscular atrophy, muscular dystrophy, muscular degeneration, myasthenia, myositis, cachexia, and senile sarcopenia.

The present disclosure (not according to the invention) also describes a food composition for improving or preventing a muscle disease, which includes oxiracetam as an active ingredient.

The present disclosure (not according to the invention) also describes a food composition for improving or preventing muscle function, which includes oxiracetam as an active ingredient.

The invention is set out in the appended set of claims.

### [Technical Solution]

To achieve the above-described purposes, the present invention provides a pharmaceutical composition for use in preventing or treating a muscle disease, which comprises oxiracetam or a pharmaceutically acceptable salt or hydrate thereof as an active ingredient, and wherein the muscle disease is any one or more selected from the group consisting of atony, muscular atrophy, muscular dystrophy, muscular degeneration, myasthenia, myositis, cachexia, and senile sarcopenia.

The present disclosure (not according to the invention) also describes a food composition for improving or preventing a muscle disease, which includes oxiracetam, or a pharmaceutically acceptable salt or hydrate thereof as an active ingredient.

The present disclosure (not according to the invention) also describes a feed additive for improving or preventing a muscle disease, which includes oxiracetam, or a pharmaceutically acceptable salt or hydrate thereof as an active ingredient.

The present disclosure (not according to the invention) also describes a use of a composition including oxiracetam, or a pharmaceutically acceptable salt or hydrate thereof as an active ingredient to prepare a pharmaceutical for preventing or treating a muscle disease.

The present disclosure (not according to the invention) also describes a method of treating a muscle disease, which includes administering a pharmaceutical composition including oxiracetam, or a pharmaceutically acceptable salt or hydrate thereof as an active ingredient into a patient with a muscle disease.

The inventors of the present invention evaluated the oxiracetam effects in 1) a dexamethasone-induced muscular atrophy animal model, 2) an aging animal model, 3) a Duchenne muscular dystrophy animal model, 4) a Cachexia muscular atrophy cell model, and 5) inflammatory muscular atrophy cell model, and confirmed the efficacy of preventing or treating a muscle disease by oxiracetam administration.

The present invention provides a pharmaceutical composition for use in preventing or treating a muscle disease, which comprises oxiracetam or a pharmaceutically acceptable salt or hydrate thereof as an active ingredient, and wherein the muscle disease is any one or more selected from the group consisting of atony, muscular atrophy, muscular dystrophy, muscular degeneration, myasthenia, myositis, cachexia, and senile sarcopenia. The present disclosure (not according to the invention) describes a pharmaceutical composition for animals for improving or preventing a muscle disease, which includes oxiracetam, or a pharmaceutically acceptable salt or hydrate thereof as an active ingredient; a food composition for improving or preventing a muscle disease, which includes oxiracetam, or a pharmaceutically acceptable salt or hydrate thereof as an active ingredient; a feed additive for improving or preventing a muscle disease, which includes oxiracetam, or a pharmaceutically acceptable salt or hydrate thereof as an active ingredient; or a method of treating a muscle disease, which includes applying oxiracetam, or a pharmaceutically acceptable salt or hydrate thereof to a human or an animal other than a human.

The present disclosure (not according to the invention) also describes a pharmaceutical composition for promoting muscle differentiation, regenerating a muscle or strengthening a muscle, which includes oxiracetam or a pharmaceutically acceptable salt thereof as an active ingredient.

The present disclosure (not according to the invention) further describes a use in therapy of a composition including oxiracetam, or a pharmaceutically acceptable salt or hydrate thereof as an active ingredient.

The present disclosure (not according to the invention) describes a novel use of oxiracetam or a pharmaceutically acceptable salt thereof to prepare a pharmaceutical for treating a muscle disease or a pharmaceutical for animals.

The oxiracetam of the pharmaceutical composition for use according to the present invention is a nootropic of the racetam family, and its Cas Number is 62613-82-5, its molecular formula is C₆H₁₀N₂O₃, its molecular weight is 158.155 g/mol, and the structure is the same as Formula 1 below.

The IUPAC name for oxiracetam is (RS)-2-(4-hydroxy-2-oxopyrrolidin-1-yl)acetamide. Oxiracetam is a white solid. A method of obtaining the oxiracetam is not particularly limited, and the oxiracetam may be chemically synthesized using a known method, or being commercially available.

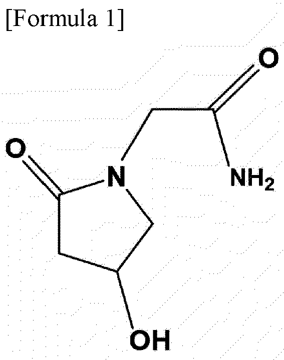

In the present invention, a nootropic is the term that refers to drugs, supplements and others that improve cognitive function, executive function, memory, etc. in a healthy individual. Racetam is generally modulate central neurotransmitters including acetylcholine and glutamate, and considered to improve memory through interaction with a glutamate receptor. Oxiracetam, which is known as a piracetam derivative, is known to be a safe substance even when ingested in high doses for a long time, and has been used in the treatment of memory disorder, cognitive disorder, cerebrovascular disorder, and cerebral infarct dementia since it easily passes through the blood-brain barrier with high selective permeability. However, there are no studies on muscular atrophy or hereditary muscular dystrophy, particularly, sarcopenia caused by aging or Duchenne muscular dystrophy (DMD), and cachexia caused by cancer or an anticancer drug.

The pharmaceutical composition for use of the present invention includes oxiracetam or a pharmaceutically acceptable salt thereof as an active ingredient, promotes muscle differentiation by increasing ATP activity required for myocytes or myoblasts to differentiate into myotubes, has a protective effect on myotubes atrophied by TNF-α, promotes grip strength improvement in a dexamethasone-induced animal model and an aging animal model, induces muscle loss inhibition and recovery mass of the tibialis anterior muscle, and promotes muscle regeneration or grip strengthening and inhibits muscular atrophy or muscle loss by increasing the recovery of lean body mass and the gastrocnemius muscle. And therefore, it was confirmed that the pharmaceutical composition of the present invention has the efficacy of improving, preventing or treating a muscle disease by muscle wasting or degeneration by administration of oxiracetam.

In addition, as a result of evaluating a muscle size, a muscle strength and muscle movement by oral administration of oxiracetam in a DMD-induced animal model, it was confirmed that the pharmaceutical composition of the present invention exhibits the efficacy of improving, preventing or treating muscular dystrophy by the oral administration of oxiracetam.

In addition, as an evaluation result obtained by administering oxiracetam to a cell model with cachexia induced by an anticancer agent, it was confirmed that the pharmaceutical composition of the present invention can inhibit the atrophy of myotube in a concentration dependent manner, and thus it was confirmed that the pharmaceutical composition of the present invention has efficacy of inhibiting, improving, preventing or treating the overall muscle loss of the body caused by secondary muscle diseases such as cancer or an anticancer agent, including sarcopenia and muscular dystrophy.

From the above-described result, it can be seen that oxiracetam, which is the active ingredient of the pharmaceutical composition for use according to the present invention, or a pharmaceutically acceptable salt or hydrate thereof are for use in the prevention or treatment of a muscle disease.

In the present invention, the muscle disease is any one or more selected from the group consisting of atony, muscular atrophy, muscular dystrophy, muscular degeneration, myasthenia, cachexia, myositis, and sarcopenia, preferably, any one or more selected from the group consisting of sarcopenia, muscular dystrophy, cachexia, and/or myositis, and more preferably, any one or more selected from the group consisting of sarcopenia, muscular dystrophy and cachexia.

In the present invention, sarcopenia may include the range of diseases caused by the decrease in muscle function, muscle loss, muscular atrophy, muscle wasting, or muscular degeneration, including atrophic diseases of all muscles present in the body, such as the eyes and face. Specifically, the decrease in muscle function, muscle loss, muscular atrophy, muscle wasting or muscular degeneration is caused by hereditary factors, acquired factors, or aging, and preferably, sarcopenia may be characterized by progressive muscle loss, particularly, atrophy, weakness, decrease, and degeneration of skeletal or voluntary muscles. Unlike muscle diseases such as muscular dystrophy, muscular atrophy and cachexia, senile sarcopenia is a gradual decrease in skeletal muscle mass or gradual weakening of muscle density and function due to various causes according to aging, and refers to a condition in which muscle mass (lean body mass) and body functions are simultaneously reduced in addition to a direct decrease in grip strength. Accordingly, to confirm the effect of improving, preventing or treating senile sarcopenia, a therapeutic effect has to be shown in all of muscle mass, grip strength and body functions, and since the increases in grip strength and muscle mass do not necessarily coincide, a clear analysis of the three effects is required unlike other muscle diseases.

In the present invention, muscular dystrophy may be any one or more selected from the group consisting of Duchenne muscular dystrophy (DMD), Becker muscular dystrophy, Emery-Dreifuss muscular dystrophy, facioscapulohumeral muscular dystrophy, limb-girdle muscular dystrophy, type 1 myotonic dystrophy, and type 2 myotonic dystrophy.

Muscular dystrophy shows muscle necrosis in muscle biopsies and shows symptoms in which the size of muscle fibers is not uniform and muscle fibers are replaced by fat and fibrotic tissue at the site of necrosis, and thus is distinguished from other muscle diseases. Particularly, among muscular dystrophy, DMD is clearly distinguished from a general muscle disease because it is caused by the mutation of a specific gene. For treatment of muscular dystrophy, as a DMD treatment guideline, a standard therapy using corticosteroids, including prednisone and deflazacort, (corticosteroid therapy) has been suggested, but it only shows the delay in symptoms, and a complete treatment is impossible. The corticosteroids used for muscular dystrophy are rarely used as a therapeutic agent because side effects occur when used for other muscle diseases. Specifically, corticosteroids induce myopathy caused by the weakness of muscle fibers and atrophy, and steroids are known to even induce myopathy when used in patients with cachexia, malnutrition, or other muscle diseases such as polymyositis. Accordingly, it can be seen that the muscular dystrophy, specifically, DMD, is distinguished from other muscle diseases in terms of not only a cause but also a therapeutic method.

In the present invention, cachexia may be characterized by a high level of general prostration symptoms which can be shown in the late stages of cancer, tuberculosis, heart failure, kidney failure, diabetes, and acquired immunodeficiency syndrome (AIDS).

In the present invention, cachexia may be cachexia caused by cancer or an anticancer agent. Cachexia refers to a condition of muscle loss due to the increase in inflammatory reactions caused by various cytokines or catabolic reactions caused by the changes in carbohydrate, protein and fat metabolisms although age-related sarcopenia or a weight loss is caused by temporary fasting, and is characterized by muscle and weight losses despite normal food intake. Muscle loss in cachexia is caused by an abnormality in muscle protein synthesis. The abnormality in muscle protein synthesis occurs because an increase in protein catabolism and a decrease in protein production due to the overactivation of various cytokines affect insulin and testosterone, which regulate muscle metabolism. However, there is no drug that shows an obvious effect yet.

The cancer is any one or more selected from the group consisting of leukemia, lymphoma, myeloma, myelodysplastic syndrome, breast cancer, head and neck cancer, esophageal cancer, stomach cancer, colorectal cancer (colon cancer), rectal cancer, anal cancer, hepatocellular liver cancer, bile duct cancer, gallbladder cancer, lung cancer (non-small cell lung cancer, small cell lung cancer), thymus cancer, kidney cancer, bladder cancer, prostate cancer, testicular cancer, ovarian cancer, cervical cancer, sarcoma, gastrointestinal stromal tumor (GIST), cancer of unknown primary site, mesothelioma, melanoma, neuroendocrine tumor, skin cancer, and blood cancer, and the anticancer agent may be any one or more selected from the group consisting of doxorubicin, irinotecan, paclitaxel, daunorubicin, docetaxel, cisplatin, and 5-fluorouracil.

Further, it was confirmed that oxiracetam, which is the active ingredient of the pharmaceutical composition for use according to the present invention, has a significant effect in animal models with DMD and senile sarcopenia, and suppresses muscular atrophy caused by an anticancer agent and restores it to a normal level or more in a cachexia cell model, resulting in an excellent improvement, prevention or therapeutic effect on all of DMD, cachexia and senile sarcopenia of different mechanisms. That is, oxiracetam, which is the active ingredient of the pharmaceutical composition for use according to the present invention, has a significant meaning in that it is possible to improve, prevent and treat three muscle diseases with one drug, and is possible to treat all inflammatory muscle diseases that can be caused by TNF-α.

In the present invention, "muscle" comprehensively refers to muscle, ligament, and tendon, and "muscle function" refers to the ability to exert a force by contraction of a muscle, and includes grip strength, which is the ability of a muscle to exert its maximum contractile force in order to overcome resistance, muscle endurance, which is the ability of a muscle to repeat contractions and relaxations for how long or how many times at a given weight, and the ability to exhibit a strong force in a short time. Such muscle function is proportional to muscle mass, and muscle function improvement means a further increase in muscle function, and more preferably, promotion of muscle differentiation, muscle regeneration, grip strengthening, or enhancement of exercise ability.

In the present invention, the promotion of muscle differentiation is to induce or cultivate differentiation into myotubes and muscle fibers, and particularly, to induce differentiation into myotubes and muscle fibers from myoblasts, and may be measured by an increase in mitochondrial activity for muscle cells and an increase in myofiber diameter caused by an increase in all myofibers. Specifically, the promotion of muscle differentiation means that the mitochondrial activity measured with an ATP detection reagent is increased 10%, 20%, 30%, 40%, 50%, 60%, 70% or more compared to the normal control group, and the diameter of a myotube fiber is increased 10%, 20%, 30%, 40% or more compared to the atrophy model in which muscular atrophy is caused by TNF-α or the diameter of a myotube fiber in the normal control is in the range of 60% to 120%, and 70% to 110%.

The myoblast of the present invention is a muscle cell in an undifferentiated state, and when a satellite cell, which is a mononuclear cell, is activated, it proliferates into a myoblast. When myoblast differentiation progresses, myoblasts fuse with other cells to form multinuclear, long and thin myotubes, thereby forming a muscle fiber. Myogenesis refers to all types of fusion of myoblasts to create myotubes, and the differentiation-promoting effect of myoblasts may be induced in skeletal muscle, cardiac muscle and smooth muscle.

The muscle regeneration and strengthening means that the diameter of tibialis anterior (TA) is increased 1%, 5%, 10%, 15%, 20%, or more compared to animals in which muscle reduction is induced by dexamethasone, the lean body mass of the gastrocnemius muscle (GS) and the lean body mass of the tibialis anterior (TA) are increased 2%, 4%, 5%, 7%, 8%, 10%, or more, all of which are measured by dual-energy X-ray absorptiometry (DEXA). In addition, the muscle regeneration and strengthening means that grip strength is increased 2%, 4%, 5%, 7%, 8%, 10%, 15%, 20%, or more compared to an animal in which muscle reduction is induced by dexamethasone, and speed is increased 0.1%, 0.5%, 0.8%, 1%, or more compared to an animal in which muscle reduction is induced by dexamethasone in an exercise stress test.

The "prevention" used herein refers to all actions of inhibiting or delaying the occurrence and symptoms of the muscle disease. The "treatment" used herein refers to all actions involved in improving or beneficially changing symptoms by the muscle disease. The "improvement" refers to all actions involved in alleviating or beneficially changing a certain symptom, disease or condition, and for example, to alleviate symptoms such as muscle reduction or muscle loss, a decrease in muscle function or exercise performance, and a decrease in ability to recover from physical fatigue, suppress pseudohypertrophy of muscles caused by muscular dystrophy, increase muscle mass, inhibit fat accumulation, enhance muscle size, grip strength, muscle function and muscle movement, or inhibit muscle loss and muscular atrophy caused by an anticancer agent or cancer.

The oxiracetam of the pharmaceutical composition for use according to the present invention may be used in the form of a pharmaceutically acceptable salt, and as a salt, an acid addition salt formed by a pharmaceutically acceptable free acid is useful. Acid addition salts may be obtained from inorganic acids such as hydrochloric acid, nitric acid, phosphoric acid, sulfuric acid, hydrobromic acid, hydroiodic acid, nitrous acid or phosphorous acid, and non-toxic organic acid such as aliphatic mono- and di-carboxylates, phenyl-substituted alkanoates, hydroxy alkanoates and alkandioates, aromatic acids, and aliphatic and aromatic sulfonic acids. Such pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylaceate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2-sulfonate, or mandelate.

The acid addition salt according to the present invention may be prepared by a conventional method, for example, by dissolving the oxiracetam in an excess aqueous acid solution and precipitating the salt in a water-miscible organic solvent, for example, methanol, ethanol, acetone or acetonitrile. Such an acid may also be prepared by heating equal amounts of oxiracetam and an acid in water or an alcohol, and drying the mixture through evaporation or suction-filtering a precipitated salt.

In addition, a pharmaceutically acceptable metal salt may be made using a base. An alkali metal or alkali earth metal salt may be obtained by, for example, dissolving a compound in an excess alkali metal hydroxide or alkali earth metal hydroxide solution, filtering an undissolved compound salt, and evaporating and drying the filtrate. Here, it is pharmaceutically suitable that a sodium, potassium or calcium salt is prepared as a metal salt. In addition, the corresponding silver salt is obtained by reacting an alkali metal or alkaline earth metal salt with a suitable silver salt (e.g., silver nitrate). In addition, the oxiracetam of the pharmaceutical composition for use according to the present invention includes all salts, hydrates and solvates that can be prepared by conventional methods, as well as pharmaceutically acceptable salts.

The addition salt according to the present invention may be prepared by a conventional method, and for example, by dissolving oxiracetam in a water-miscible organic solvent, for example, methanol, ethanol, acetone or acetonitrile, adding an excess organic acid or inorganic acid aqueous solution, and then performing precipitation or crystallization. Subsequently, after evaporating the solvent or excess acid from the mixture, the resulting product is dried to obtain, thereby obtaining the addition salt, or the addition salt may be obtained by suction filtration of a precipitated salt.

The "hydrate" used herein refers to the oxiracetam of the pharmaceutical composition for use according to the present invention or a salt thereof, which includes a stoichiometric or non-stoichiometric amount of water bound by a non-covalent intermolecular force. The hydrate of the oxiracetam of the pharmaceutical composition for use according to the present invention may contain a stoichiometric or non-stoichiometric amount of water bound by a non-covalent intermolecular force. The hydrate may contain 1 equivalent or more, preferably, 1 to 5 equivalents of water. Such a hydrate may be prepared by crystallizing the oxiracetam of the pharmaceutical composition for use according to the present invention, an isomer thereof, or a pharmaceutically acceptable salt thereof from water or a water-containing solvent.

The "solvate" used herein refers to a compound of the pharmaceutical composition for use according to the present invention or a salt thereof, which includes a stoichiometric or non-stoichiometric amount of solvent bound by a non-covalent intermolecular force. Preferable solvents are volatile, non-toxic, and/or suitable for administration to humans.

The "isomer" used herein refers to a compound of the pharmaceutical composition for use according to the present invention or a salt thereof, which has the same chemical or molecular formula, but is structurally or sterically different. Such isomers include structural isomers such as a tautomer, stereoisomers such as an R or S isomer having an asymmetric carbon center, or a geometric isomer (trans or cis), and enantiomers.

The "pharmaceutical composition", "pharmaceutical", "pharmaceutical composition for animals" or "pharmaceutical for animals" may further include suitable carriers, excipients and diluents conventionally used in the preparation of a pharmaceutical composition, in addition to oxiracetam or a pharmaceutically acceptable salt thereof as an active ingredient.

The "carrier" is a compound that facilitates the addition of the compound into cells or tissue. The "diluent" is a compound not only stabilizing a biologically active form of a target compound but also dissolving the compound in water.

As the carrier, excipient and diluent, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, methylhydroxy benzoate, propylhydroxybenzoate, talc, magnesium stearate, an ion exchange resin, alumina, aluminum stearate, lecithin, a serum protein (e.g., human serum albumin), a buffer (e.g., various types of phosphates, glycine, sorbic acid, potassium sorbate, partial glyceride mixture of saturated vegetable fatty acids), water, a salt, an electrolyte (e.g., protamine sulfate, disodium hydrogen phosphate, potassium hydrogen phosphate, sodium chloride, and a zinc salt), colloidal silica, magnesium trisilicate, polyethylene glycol, polyacrylate, wax, wool grease and mineral oil may be used.

The pharmaceutical composition, pharmaceutical, pharmaceutical composition for animals, or pharmaceutical for animals may be used in an oral formulation such as a powder, a granule, a tablet, a capsule, a suspension, an emulsion, a syrup or an aerosol, an external preparation, a suppository, or a sterile injection solution. Specifically, in preparation, the pharmaceutical composition, pharmaceutical, pharmaceutical composition for animals, or pharmaceutical for animals may use a generally used diluent or excipient, for example, a filler, a thickening agent, a binder, a wetting agent, a disintegrant or a surfactant. Solid preparations for oral administration include a tablet, a pill, a powder, a granule, and a capsule, and such solid preparations may be formulated by mixing the pharmaceutical composition for use of the present invention with at least one or more excipients, for example, starch, calcium carbonate, sucrose, lactose, and gelatin. In addition, in addition to a simple excipient, lubricants such as magnesium stearate and talc may also be used. Liquid preparations for oral administration may include a suspension, an oral liquid, an emulsion, and a syrup, and contain various excipients, for example, a wetting agent, a sweetening agent, a fragrance, and a preservative, in addition to water or liquid paraffin, which is a frequently used simple diluent. Preparations for parenteral administration include a sterile aqueous solution, a non-aqueous solvent, a suspension, an emulsion, a freeze-dried preparation and a suppository. As the non-aqueous solvent and suspension, propylene glycol, polyethylene glycol, a vegetable oil such as olive oil, an injectable ester such as ethyl oleate, Hank's solution, or Ringer's solution may be used. As a suppository base, Witepsol, Macrogol, Tween 61, cacao butter, laurinum, or glycerogelatin may be used.

Administration routes for the pharmaceutical composition, pharmaceutical, pharmaceutical composition for animals, or pharmaceutical for animals include oral, intravenous, intramuscular, intraarterial, intramedullary, intrathecal, intracardiac, transdermal, subcutaneous, intraperitoneal, intranasal, enteral, local, sublingual and rectal administrations. Oral or parenteral administration is preferable. The term "parenteral" used herein means subcutaneous, intravenous, intramuscular, intraarticular, intrabursal, intrasternal, intrathecal, intralesional, intracranial injections or infusions. The pharmaceutical composition for use of the present invention may be administered in the form of a suppository for rectal administration.

The pharmaceutical composition for use of the present invention may be formulated as a preparation for oral administration or parenteral administration via the above-described administration route. When formulating, one or more buffers (e.g., saline or PBS), antioxidants, bacteriostatic agents, chelating agents (e.g., EDTA or glutathione), fillers, bulking agents, binders, adjuvants (e.g., aluminum hydroxide), suspending agents, thickening agents, wetting agents, disintegrants or surfactants, diluents or excipients may be used.

Solid preparations for oral administration include a tablet which contains nanoparticles (preparations for increasing a bioabsorption rate and rapidly exhibiting an effect), pill, powder, granule, liquid, gel, syrup, slurry, suspension or capsule, , and the solid preparations may be formulated by mixing the pharmaceutical composition for use of the present invention with at least one or more excipients, such as starch (corn starch, wheat starch, rice starch, or potato starch), calcium carbonate, sucrose, lactose, dextrose, sorbitol, mannitol, xylitol, erythritol, maltitol, cellulose, methyl cellulose, sodium carboxymethylcellulose, hydroxypropylmethylcellulose or gelatin. For example, tablets or sugar-coated pills may be obtained by mixing an active ingredient with a solid excipient, pulverizing the mixture, adding a suitable additive, and processing the resulting mixture into a granulated mixture.

In addition to simple excipients, lubricants such as magnesium stearate and talc are also used. Liquid formulations for oral administration may include a suspension, an oral liquid, an emulsion, and a syrup, and contain various excipients, for example, a wetting agent, a sweetening agent, a fragrance, and a preservative, in addition to water or liquid paraffin, which is a frequently used as simple diluent.

In addition, in some cases, cross-linked polyvinylpyrrolidone, agar, alginic acid or sodium alginate may be added as a disintegrant, and an anti-aggregating agent, a lubricant, a wetting agent, a fragrance, an emulsifier, and a preservative may be further included.

In the case of parenteral administration, the pharmaceutical composition for use of the present invention may be formulated in the form of an injection, a transdermal preparation, and a nasal inhalant, together with a suitable parenteral carrier, by a method known in the art. The injection must be sterilized and protected from contamination with microorganisms such as bacteria and fungi. Suitable carriers for injections may be water, ethanol, a polyol (e.g., glycerol, propylene glycol, or liquid polyethylene glycol), a mixture thereof, and/or a solvent or dispersing medium containing vegetable oil. More preferably, as suitable carriers, Hank's solution, Ringer's solution, phosphate buffered saline (PBS) containing triethanolamine, or an isotonic solution such as sterile water for injection, 10% ethanol, 40% propylene glycol, or 5% dextrose, may be used. To protect the injection from microbial contamination, various antibacterial and antifungal agents such as paraben, chlorobutanol, phenol, sorbic acid, and thimerosal may be further included. In addition, the injection may, in most cases, further include, isotonic agents such as sugar or sodium chloride.

Preparations for transdermal administration are formulated as an ointment, a cream, a lotion, a gel, a topical liquid, a paste, a liniment, and an aerosol. The "transdermal administration" refers to the delivery of an effective amount of active ingredient contained in a pharmaceutical composition by topically administering the pharmaceutical composition to the skin.

In the case of an inhalant, the compound used in the pharmaceutical composition for use according to the present invention may be conveniently delivered in the form of a pressurized pack or nebulizer, or an aerosol spray using a suitable propellant, for example, dichlorofluoromethane, trichlorofluoromethane, dichlorotetrafluoroethane, carbon dioxide, or other suitable gases. In the case of a pressurized aerosol, a dosage unit may be determined by providing a valve delivering a measured amount. For example, a gelatin capsule and cartridge used for an inhalant or nebulizer may be formulated to contain a powder mixture of a compound, and a suitable powder base such as lactose or starch. Formulations for parenteral administration are described in all recipes commonly known in pharmaceutical chemistry (Remington's Pharmaceutical Science, 15th Edition, 1975. Mack Publishing Company, Easton, Pennsylvania 18042, Chapter 87: Blaug, Seymour).

A dosage of the pharmaceutical composition, pharmaceutical, pharmaceutical composition for animals, or pharmaceutical for animals may vary according to the age, sex, or body weight of a patient or an animal to be treated, and may depend on, among other things, the condition of a subject to be treated, a specific category or type of a disease to be treated, an administration route, and the property of a therapeutic agent to be used.

The pharmaceutical composition, pharmaceutical, pharmaceutical composition for animals, or pharmaceutical for animals is appropriately selected according to the absorption rate of an active ingredient in the body, and may be generally administered daily at 0.1 to 1,000 mg/kg, preferably 1 to 500 mg/kg, more preferably 5 to 250 mg/kg, and most preferably 10 to 100 mg/kg. The unit dosage-form preparation formulated as described above may be administered several times at regular intervals as needed.

The pharmaceutical composition, pharmaceutical, pharmaceutical composition for animals, or pharmaceutical for animals may be administered individually as a prophylactic or therapeutic agent or administered in combination with another therapeutic agent, or may be administered sequentially or simultaneously with a conventional therapeutic agent.

The treatment method for the muscle disease is to administer the composition to a human, or a non-human animal, particularly, a mammal, and may be specifically oral administration. For example, the treatment method for the muscle disease is to orally administer the composition to a subject with a muscle disease.

The subject with a muscle disease may have any one or more muscle diseases selected from the group consisting of atony, muscular atrophy, muscular dystrophy, muscular degeneration, myasthenia, myositis, cachexia, and senile sarcopenia as described above. The subject with a muscle disease may have senile sarcopenia in which muscle mass, and muscle density and functions are progressively decreased by aging, may be a case in which all of muscle mass, and grip strength and body functions have declined, a case in which a decrease in muscle function, muscle reduction, muscular atrophy, muscle wasting, or muscular degeneration is induced by a specific genetic mutation, or a case suffering from cachexia that causes a decline in muscle function, muscle reduction, muscular atrophy, or muscle wasting occurs by cancer or an anticancer agent administered to treat the cancer.

For the dosage, administration method, and frequency of administration for the treatment, reference may be made to the dosage, administration method and administration frequency of the pharmaceutical composition, pharmaceutical, pharmaceutical composition for animals, or pharmaceutical for animals.

The present disclosure (not according to the invention) relates to a food composition for improving or preventing a muscle disease, which includes oxiracetam, or a pharmaceutically acceptable salt or hydrate thereof as an active ingredient.

The present disclosure (not according to the invention) relates to a food composition for improving muscle function, which includes oxiracetam, or a pharmaceutically acceptable salt or hydrate thereof as an active ingredient.

For the food composition (not according to the invention), details of the oxiracetam or a pharmaceutically acceptable salt thereof have been described above.

The food composition (not according to the invention) may be used in improvement or prevention of any one or more muscle diseases selected from the group consisting of atony, muscular atrophy, muscular dystrophy, muscular degeneration, myasthenia, cachexia, myositis, and sarcopenia. For the food composition (not according to the invention), details of the muscle diseases have been described above.

The athletic ability used herein is also referred to as exercise performance ability, and the ability to perform physical movements seen in daily life or sports when the movements are classified by appearance into running, jumping, throwing, and swimming. The improvement in athletic ability refers to an improvement or enhancement in exercise performance, and specifically, refers to the improvement or enhancement of the movement rapidly, strongly, and accurately for a long time, or restoration to the original athletic ability or to a better degree than the original when the athletic ability has declined. The athletic ability may be defined by parameters such as grip strength, agility and endurance.

The food composition (not according to the invention) may include all forms of functional food, nutritional supplements, health functional food, food additives and feed, and is intended to be eaten by animals including humans or livestock. The type of food composition may be prepared in various forms according to conventional methods known in the art.

The "food composition" includes food ingredients that can be used as food listed in the Standards and Specifications ("Food Code") for food normally used in food manufacturing, and food additives listed in the food additive code, in addition to oxiracetam or a pharmaceutically acceptable salt thereof as an active ingredient.

The food composition (not according to the invention) includes, for example, proteins, carbohydrates, lipids, nutrients, seasonings, and flavoring agents. The carbohydrates may include monosaccharides such as glucose and fructose; disaccharides such as maltose, sugar and lactose; oligosaccharides or polysaccharides, such as dextrin, starch syrup and cyclodextrin; and sugar alcohols such as xylitol, sorbitol and erythritol. The flavoring agents may include natural flavoring agents [thaumatin, stevia extract (e.g., rebaudioside A and glycyrrhizin)], and synthetic flavoring agents (saccharin and aspartame).

When the food composition (not according to the invention) is prepared using the oxiracetam or a pharmaceutically acceptable salt thereof as an active ingredient, the oxiracetam or a pharmaceutically acceptable salt thereof may be included, for example, at a content exhibiting efficacy on a muscle disease, for example, 0.1 to 99 wt%, 0.5 to 95 wt%, 1 to 90 wt%, 2 to 80 wt%, 3 to 70 wt%, 4 to 60 wt%, or 5 to 50 wt%.

In the food composition (not according to the invention), the active ingredient, that is, the oxiracetam or a pharmaceutically acceptable salt thereof may vary according to the condition or weight of a food ingester, the presence, severity or extent of a disease, but may be suitably selected by one of ordinary skill in the art. For example, based on a daily dose, the active ingredient may be contained at 1 to 5,000 mg, 5 to 2,000 mg, 10 to 1,000 mg, 20 to 800 mg, or 25 to 500 mg, and the administration frequency may be adjusted by those of ordinary skill in the art within the range of three times a day to once a week without particular limitation. In the case of long-term ingestion for health and hygiene or for heal control, the amount may be lower than the above range.

The food composition (not according to the invention) may be prepared in the form of, for example, a powder, a granule, a tablet, a capsule, a pill, an extract, a jelly, a tea bag, or a beverage.

In addition, to provide functionality for prevention or improvement of a muscle disease and improvement of muscle function to general food, the oxiracetam or a pharmaceutically acceptable salt thereof may be added, and addible food may be added to, for example, confectioneries, bread or rice cakes, cocoa processed products or chocolates, edible meat or egg products, processed fish meat products, tofu or jelly products, noodles, teas, coffee, beverages, special purpose foods, soy sauce, seasonings, dressings, kimchi, salted fish, pickles, stewed foods, alcoholic beverages, raisins, other foods, etc., exemplified in the Food Standards and Specifications ('Food Code') in accordance with Article 7 of the Food Sanitation Act. In addition, it may be added to dairy products, processed meat products, packaged meats, and processed egg products, exemplified in the Processing Standards and Ingredient Specifications for Livestock Products in accordance with Article 4 of the Livestock Products Sanitation Control Act ('Livestock Products Code').

Meanwhile, the food composition containing the oxiracetam or a pharmaceutically acceptable salt thereof as an active ingredient may be used as a "health functional food" that helps prevent or improve a "muscle disease," or may be used as a "health functional food that helps muscle functions".

The term "health functional food" refers to food (Article 3 Paragraph 1 of the Act on Health Functional Food) manufactured (including processing) according to legal standards using raw materials or ingredients useful for the human body. The "health functional food" may have differences in terms or ranges between countries, but may be an American 'Dietary Supplement,' European 'Food Supplement,' Japanese 'health Functional Food' or 'Food for Special Health Use (FoSHU)', or Chinese 'Health Food'.

The food composition or health functional food may additionally contain a food additive, and unless otherwise specified, the suitability as a food additive follows the Specifications and Standards for relevant items in accordance with the general provisions and general test method of the Korean Food Additives Code.

In addition, the health functional food may use a health functional food material associated with improvement in exercise performance capability or grip strength, such as *Hovenia dulcis* extract powder, Cordyceps fermented extract, creatine, or *Schisandra chinensis* extract as a raw material reported as a 'functional raw material' used in "health functional food help prevent or improve a muscle disease" or "health functional food helping muscle function" or an individually recognized raw material, in addition to the oxiracetam or a pharmaceutically acceptable salt thereof.

It is described herein (not according to the invention), that the composition of the present invention may be added to a feed additive or a feed composition including the same to prevent or improve a muscle disease.

The present disclosure (not according to the invention) describes a feed composition for improving muscle function, which includes oxiracetam or a pharmaceutically acceptable salt thereof as an active ingredient.

The term "feed additive" used herein includes a material added to feed for the purpose of various effects such as nutrient supplementation and prevention of weight loss, enhancement of digestibility of fibers in feed, improvement of oil quality, prevention of a reproductive disorder and improvement of birth rate, and prevention of high temperature stress in summer. The feed additive of the present disclosure (not according to the invention) corresponds to an auxiliary feed under the Feed Management Act, and further includes a mineral preparation such as sodium bicarbonate, bentonite, magnesium oxide or a complex mineral, a mineral preparation, which is a trace mineral, such as zinc, copper, cobalt or selenium, a vitamin such as keratin, vitamin A, D or E, nicotinic acid, vitamin B complex, a protective amino acid preparation such as methionine or lysine, a protective fatty acid preparation such as a fatty acid calcium salt, or a live bacterial or yeast preparation such as a probiotic (lactic acid bacterial product), a yeast culture or a mold fermented product.

The term "feed" used herein is any natural or artificial diet or meal, or an ingredient of the meal, which is for or suitable for being eaten, ingested and digested by all animals including pets, and feed including the composition for preventing or improving a muscle disease as described herein as an active ingredient can be prepared in various types of feed known in the art, and preferably includes concentrated feed, coarse fodder and/or special-purpose feed.

The concentrated feed includes seed fruits including grains, such as wheat, oats and corn; bran including rice bran, wheat bran or barley bran as a by-product obtained by purification of grains; residues including expeller cakes, which are by-products from beans, rapeseed, sesame seed, flaxseed or coconut by pressing, and residual starches, which are main ingredients of starch residues obtained by extracting starch from sweet potatoes or potatoes; fish powder, fish residues, or fish solubles, which are concentrates of fresh liquid obtained from fish; animal feed such as meat powder, blood powder, feather meal powder, skim milk powder, skim milk powder, or dried whey obtained by drying whey which is a residual liquid when producing cheese from milk and casein from skim milk; yeasts; chlorella; and seaweed.

The coarse fodder includes grass feed such as wild grass, pasturage or soiling; root & bulb vegetables such as turnips for feed, beets for feed, or rutabaga, which is one turnip; silage, which is storage feed in which grass, soiling crops, grains & fruits are filled in silos and fermented with lactic acid; hay obtained by drying cut wild grass and pasturage, straw for breeding crops; and legume leaves. The special-purpose feed includes mineral feed such as oyster shells or rock salt; urea feed such as urea or diureide isobutane, which is a derivative thereof; feed additives, which are materials added to blended feed in a small amount in order to supplement an ingredient that is likely to be insufficient when blending only a natural feed raw material or increase the storability of feed; and dietary supplements.

The feed additive for preventing or improving a muscle disease according to the present disclosure (not according to the present invention) can be produced by adding oxiracetam in an appropriate effective concentration range according to various feed preparation methods known in the art.

The feed additive according to the present disclosure (not according to the present invention) can be applied to a subject for the purpose of preventing or improving a muscle disease without limitation. For example, it can also be applied to any subject selected from non-human animals such as cattle, horses, pigs, goats, sheep, dogs, cats, rabbits, birds, fish, etc.

### [Advantageous Effects]

In the present disclosure (not according to the present invention), oxiracetam can prevent, treat or improve a muscle disease, and particularly, by confirming an improvement, prevention or treatment effect in a dexamethasone-caused muscular atrophy animal model, an aging-induced animal model, a DMD animal model and a cachexia cell model, it can be used as a pharmaceutical or food composition for preventing or treating a muscle disease.

Specifically, the oxiracetam of the present disclosure (not according to the present invention) can significantly increase grip strength, a muscle diameter and muscle mass, and exhibit an effect of increasing muscle differentiation, muscle regeneration and muscle mass in a muscular atrophy animal model, an aging-induced animal model, and a genetic muscular dystrophy animal model, so it can be used as a pharmaceutical or food composition for improving muscle function.

### [Description of Drawings]

FIG. 1 is a graph showing the results of measuring grip strengths of a normal group and dexamethasone-treated group.
FIG. 2 is a set of representative images of a normal group and dexamethasone-treated group analyzed by dual energy X-ray absorptiometry (DEXA) on day 21.
FIG. 3 is a graph showing the results of measuring a tibialis anterior muscle size (T1) in a normal group and dexamethasone-treated group on day 21.
FIG. 4 is a graph showing the results of measuring total lean body mass in a normal group and dexamethasone-treated group on day 21.
FIG. 5 is a graph showing the results of measuring a tibialis anterior muscle size (T1) in a dexamethasone-treated group (dexamethasone, excipient) and an oxiracetam-administered group of dexamethasone-treated muscle disease model animals.
FIG. 6 is a graph showing the results of measuring the lean body mass of the gastrocnemius muscle (GS) in a dexamethasone-treated group (dexamethasone, excipient) and an oxiracetam-administered group of dexamethasone-treated muscle disease model animals.
FIG. 7 is a graph showing the results of measuring the lean body mass of the tibialis anterior (TA) in a dexamethasone-treated group (dexamethasone, excipient) and an oxiracetam-administered group of dexamethasone-treated muscle disease model animals.
FIG. 8 is a graph showing the results of measuring grip strengths in a dexamethasone-treated group (dexamethasone, excipient) and an oxiracetam-administered group of dexamethasone-treated muscle disease model animals.
FIG. 9 is a graph showing the results of an exercise stress test for muscle function in a dexamethasone-treated group (dexamethasone, excipient) and an oxiracetam-administered group of dexamethasone-treated muscle disease model animals.
FIG. 10 is a graph showing the change in grip strength according to a control (vehicle) and oxiracetam-administered groups (oxiracetam 30, oxiracetam 100, oxiracetam 300) of aging mice. The control (vehicle) was not administered a drug, and was a normal saline-treated group.
FIG. 11 is a set of representative images of a control (vehicle) and oxiracetam-administered groups (oxiracetam 30, oxiracetam 100, oxiracetam 300) of aging mice analyzed by dual energy X-ray absorptiometry (DEXA) on day 83 of aging mice.
FIG. 12 is a graph obtained by measuring the lean body mass of the tibialis anterior (TA) every 7 days according to a control (vehicle) and oxiracetam-administered groups (oxiracetam 30, oxiracetam 100, oxiracetam 300) of aging mice.
FIG. 13 is a graph obtained by measuring the lean body mass of the gastrocnemius muscle (GS) every 7 days according to a control (vehicle) and oxiracetam-administered groups (oxiracetam 30, oxiracetam 100, oxiracetam 300) of aging mice.
FIG. 14 is a graph showing the results of an exercise stress test for muscle function in a control (vehicle) and oxiracetam-administered groups (Oxi 30, Oxi 100, Oxi 300) of aging mice, and FIG. 14A is the measured running distance (m), and FIG. 14B is the measured running time (sec).
FIG. 15 is a graph showing the results of the change in grip strength in a control (vehicle) and oxiracetam-administered groups (Oxi 42, Oxi 83, Oxi 167, Oxi 333) of aging mice.
FIG. 16 is a set of representative images of a control (vehicle) and oxiracetam-administered groups (Oxi 42, Oxi 83, Oxi 167, Oxi 333) of aging mice analyzed by dual energy X-ray absorptiometry (DEXA) on day 91 of aging mice, and a graph showing the results of measuring the lean body mass of the gastrocnemius muscle (GS) every 7 days.
FIG. 17 is a graph showing the results of measuring the lean body mass of the tibialis anterior (TA) every 7 days in a control (vehicle) and oxiracetam-administered groups (Oxi 42, Oxi 83, Oxi 167, Oxi 333) of aging mice.
FIG. 18 is a graph showing the results of measuring the change in grip strength in a control (vehicle), a dexamethasone-treated group (dexamethasone, excipient), and drug-administered groups (oxi 30, pi 100, phenylpi 30, ani 30, prami 30, briva 30 and roli 30).
FIG. 19 is a graph showing the results of measuring the change in grip strength in a control (vehicle), a dexamethasone-treated group (dexamethasone, excipient), and drug-administered groups (oxi 30, pi 100, phenylpi 30, ani 30, leveti 30, nefi 30 and colu 30).
FIG. 20 is a graph showing the results of measuring the change in grip strength on day 21, compared to day 0, in a control (vehicle), a dexamethasone-treated group (dexamethasone, excipient), and drug-administered groups (oxi 30, rolzi 30, faso 30 and prami 30).
FIG. 21 is a graph showing the results of measuring the lean body mass of the tibialis anterior (TA) every 7 days in a control (vehicle), a dexamethasone-treated group (dexamethasone, excipient), and drug-administered groups (oxi 30, pi 100, phenylpi 30, ani 30, leveti 30, nefi 30 and colu 30).
FIG. 22 is a graph showing the results of measuring the lean body mass of the tibialis anterior (TA) every 7 days in a control (vehicle), a dexamethasone-treated group (dexamethasone, excipient), and drug-administered groups (oxi 30, pi 100, phenylpi 30, ani 30, prami 30, briva 30 and roli 30).
FIG. 23 is a graph showing the results of measuring the lean body mass of the tibialis anterior (TA) every 7 days in a control (vehicle), a dexamethasone-treated group (dexamethasone, excipient), and drug-administered groups (oxi 30, rolzi 30, faso 30 and prami 30).
FIG. 24 is a graph showing the results of measuring the lean body mass of gastrocnemius muscle (GS) every 7 days in a control (vehicle), a dexamethasone-treated group (dexamethasone, excipient), and drug-administered groups (oxi 30, pi 100, phenylpi 30, ani 30, leveti 30, nefi 30 and colu 30).
FIG. 25 is a graph showing the results of measuring the lean body mass of the gastrocnemius muscle (GS) every 7 days in a control (vehicle), a dexamethasone-treated group (dexamethasone, excipient), and drug-administered groups (oxi 30, rolzi 30, faso 30 and prami 30).
FIG. 26 is a graph showing the results of measuring the lean body mass of the gastrocnemius muscle (GS) every 7 days in a control (vehicle), a dexamethasone-treated group (dexamethasone, excipient), and drug-administered groups (oxi 30, pi 100, phenylpi 30, ani 30, prami 30, briva 30 and roli 30).
FIG. 27 is a graph showing the results of measuring the change in grip strength on day 21 compared to day 0 in a control (vehicle), a dexamethasone-treated group (dexamethasone, excipient), and drug-administered groups (Oxiracetam30, Mirtazapine10 and Olanzapine10).
FIG. 28 is a graph obtained by measuring the lean body mass of the tibialis anterior (TA) every 7 days in a control (vehicle) and oxiracetam-administered groups (Oxiracetam 30, Mirtazapine 10 and Olanzapine 10).
FIG. 29 is a graph obtained by measuring the lean body mass of the gastrocnemius muscle (GS) every 7 days in a control (vehicle) and oxiracetam-administered groups (Oxiracetam 30, Mirtazapine 10 and Olanzapine 10).
FIG. 30 is a set of images of fat distribution in a control (vehicle) and a drug-administered group (oxiracetam) captured by dual energy X-ray absorptiometry (DEXA).
FIG. 31 is a graph showing the results of measuring the total lean body mass in a control (vehicle) and a drug-administered group (oxiracetam).
FIG. 32 is a graph showing the results of measuring a body fat mass (fat, %) in a control (vehicle) and a drug-administered group (oxiracetam).
FIG. 33 is a set of images of a control (vehicle) and a drug-administered group (oxiracetam) captured by dual energy X-ray absorptiometry (DEXA).
FIG. 34 is a graph showing the results of measuring the lean body mass of the tibialis anterior (TA) in a control (vehicle) and a drug-administered group (oxiracetam).
FIG. 35 is a graph showing the results of measuring the lean body mass of the gastrocnemius muscle (GS) in a control (vehicle) and a drug-administered group (oxiracetam).
FIG. 36 is a graph showing the results of measuring the change in grip strength in a control (vehicle) and a drug-administered group (oxiracetam).
FIG. 37 is a graph showing the results of measuring the change in body weight in a control (vehicle) and a drug-administered group (oxiracetam).
FIG. 38 is a graph shown by confirming the degree of the change in ATP content in myotube cells (myotube) in an oxiracetam-treated group (oxiracetam, 10 µM) and a non-treated group (solvent-treated, control).
FIG. 39 is a set of images of TNF-α-treated myotube cells (TNF-α 10 ng/ml) in an oxiracetam-treated group (oxiracetam, 10 µM) and a non-treated group (oxiracetam 0 µM; TNF-α 10 ng/ml), captured by an optical microscope. The control is a solvent-treated group (TNF-α or oxiracetam non-treated group).
FIG. 40 is a graph showing the results of measuring the diameter of a TNF-α-treated myotube cells (TNF-α 10 ng/ml) in an oxiracetam-treated group (oxiracetam 10 µM) and a non-treated group (oxiracetam 0 µM; TNF-α, 10 ng/ml). The control is a non-treated group (TNF-α or oxiracetam non-treated group).
FIG. 41 is a set of images of an anticancer agent-treated group (doxorubicin), oxiracetam-treated groups (oxiracetam 0.01 µM, 0.1 µM, 1 µM, 10 µM and 30 µM) and a control captured by an optical microscope.
FIG. 42 is a graph showing the results of measuring the diameter of myotube cells (myotube) in an anticancer agent-treated group (doxorubicin), oxiracetam-treated groups (oxiracetam 0.01 µM, 0.1 µM, 1 µM, 10 µM and 30 µM) and a control.

### [Modes of the Invention]

Hereinafter, to help in understanding the present invention, exemplary examples will be suggested. However, it should be understood that the examples are not provided to limit the scope of the present invention and all alternatives and modifications within the scope of the accompanying claims are included in the present invention.

### <Examples>

Oxiracetam having a structure of Formula 1 was purchased from Tokyo Chemical Industry (Japan; Product No. O0398, CAS RN:62613-82-5, purity: 96%) and used.

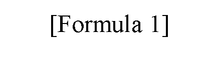

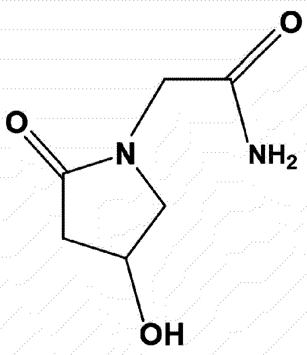

### <Experimental Examples>

### Experimental Example 1. Efficacy analysis in muscular dystrophy animal model

### 1-1. Preparation of experimental animals and samples

For an experimental group in the present invention, oxiracetam having a structure of Formula 1 was purchased from Tokyo Chemical Industry (Japan; Product No. O0398, CAS RN:62613-82-5, purity: 96%) and used.

As experimental animals, 7-week-old male ICR mice were purchased and used. Until the day of the experiment, general feed (EEGJ30060: Cargill Agri Purina, Seongnam, Korea) and water were sufficiently supplied to the experimental animals, and the animals were acclimated for 1 week under an environment of a temperature of 23 ± 2 °C, a humidity of 55 ± 10%, and a light-dark cycle of 12 hours-12 hours and used in the experiment.

After the acclimation period for the experimental animals, to create a muscular atrophy model, the mice were randomly divided into groups of 10 animals, such as a normal group (n=10), a dexamethasone-treated group (n=10), and an oxiracetam-administered group (n=10). The groups except for the normal group were intraperitoneally injected with dexamethasone at a dose of 10 mg/kg from day 7 to day 21 for 14 days. In the case of the oxiracetam-administered group, 7 days before the intraperitoneal administration of dexamethasone, oxiracetam was orally administered using a Zonde needle at a dose of 30 mg/kg/day once a day for 21 days. During the same period, the normal group was administered no drug, and 100 µL of normal saline was orally administered at a time.

**[Table 1]**

| Classification | Description |
|---|---|
| Non-treated group (normal) | Administered normal saline daily for 21 days (normal) |
| Oxiracetam-administered group (Oxiracetam) | Administered dexamethasone daily from day 7 to day 21 for 14 days (10 mg/kg/day) + Administered oxiracetam daily from day 1 to day 21 for 21 days (30 mg/kg/day) |
| Dexamethasone-treated group (Dexamethasone, excipient; CMC) | Administered dexamethasone daily from day 7 to day 21 for 14 days (10 mg/kg/day) + Administered excipient (CMC) daily from day 1 to day 21 |

### 1-2. Change in lean body mass

The lean body mass for the gastrocnemius muscle (GS) and tibialis anterior (TA) for each group was measured every 7 days. Specifically, animal models of each group were anesthetized using 3% isoflurane, fixed to a photographing bed, and then photographed. The respiration rates and temperatures of the animal models during photographing were measured using an animal monitoring system (SA Instruments, USA).

The whole body of the animal model was photographed through dual energy X-ray absorptiometry (DEXA) using iNSiGHT VET DXA (Osteosys, KR), and the lean body mass of each of the gastrocnemius muscle (GS) and the tibialis anterior (TA) was measured and calculated from the two-dimensional images of the gastrocnemius muscle (GS) and the tibialis anterior (TA) by a program using the region of interest (ROI).

### 1-3. Grip strength measurement

To measure grip strength, a grip strength test was performed. The maximum force presented when the forelimbs of an animal model of each group could no longer hold the rod by pulling the tail horizontally while holding the tail of the animal model and holding the rod of a device with both front paws was considered to be grip strength (N). The grip strength was measured once every 2 days and 11 times for each mouse.

### 1-4. Tibialis anterior diameter (T1)

Tibialis anterior diameters (T1) were measured from two-dimensional images captured by dual-energy X-ray absorptiometry (DEXA) for each group and compared.

### 1-5. Exercise stress test (treadmill test)

First, the experimental animals in each group were acclimatized to a treadmill machine twice before measurement (1^{st} round: inclination 10°, 10 minutes at 5 m/min and 10 minutes at 10 m/min; 2^{nd} round: inclination 10°, 5 minutes at 5 m/min and 15 minutes at 10 m/min). On the day of the experiment, the experimental animals were allowed to run for 20 minutes at an initial speed of 10 m/min at an inclination of 10°, and then the speed was increased by 2 m/min every 2 minutes, and then the running distance and time were measured with the end point when the experimental animal stayed for 10 seconds without running.

### 1-6. Statistical analysis

Statistical processing was performed using GraphPad Prism (GraphPad Software Inc., San Diego, CA, USA). The results of each repeated experiment were expressed as mean ± standard error of mean, and analyzed using the analysis of variance (Tukey's test), and statistical significance was acknowledged when the P-value was 0.05 or less.

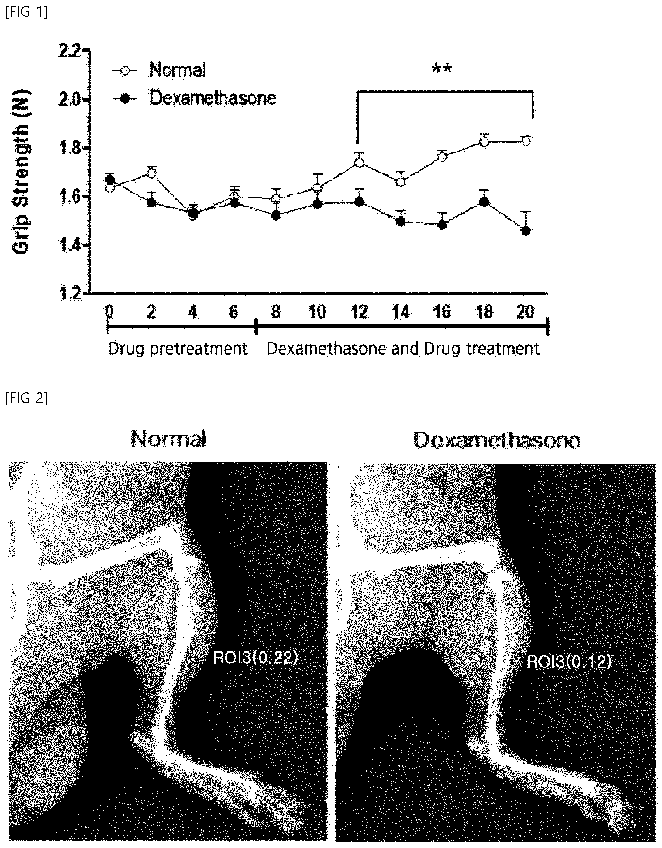

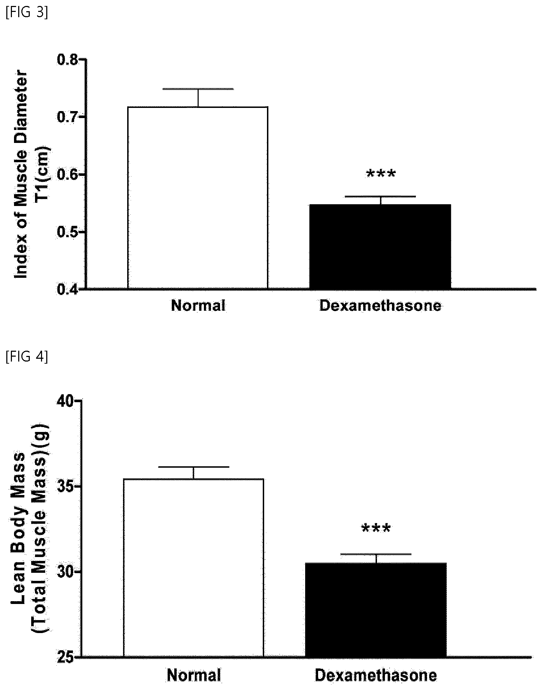

FIG. 1 is a graph showing the results of measuring grip strengths of a normal group and dexamethasone-treated group, and FIG. 2 is a set of representative images of a normal group and dexamethasone-treated group analyzed by dual energy X-ray absorptiometry (DEXA) on day 21. FIG. 3 is a graph showing the results of measuring a tibialis anterior muscle size (T1), and FIG. 4 is a graph showing the results of measuring total lean body mass on day 21 in a normal group and dexamethasone-treated group.

As shown in FIGS. 1 to 4, it was confirmed that, in the dexamethasone-treated group, 14-day dexamethasone administration led to reduced grip strength, a significant decrease in tibialis anterior diameter (T1), and a great decrease in lean body mass. That is, it can be confirmed that a muscle reduction model inducing muscular atrophy similar to senile muscular atrophy through dexamethasone administration was created.

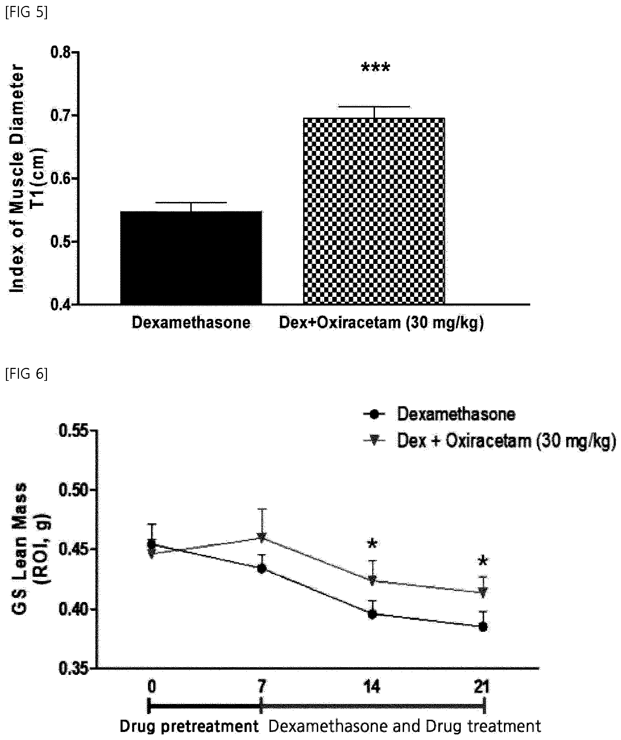

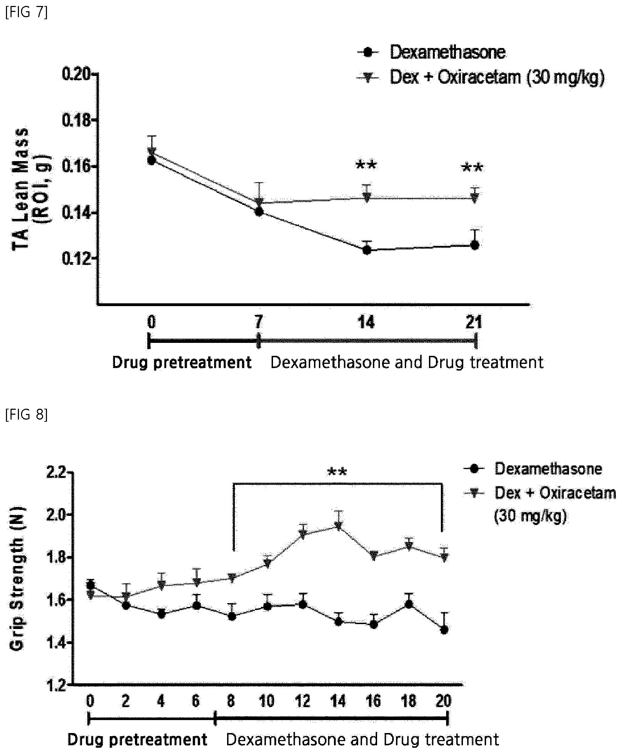

FIG. 5 is a graph showing the results of measuring a tibialis anterior muscle size (T1) in a dexamethasone-treated group (dexamethasone, excipient) and an oxiracetam-administered group, FIG. 6 is a graph showing the results of measuring the lean body mass of the gastrocnemius muscle (GS) in a dexamethasone-treated group (dexamethasone, excipient) and an oxiracetam-administered group, and FIG. 7 is a graph showing the results of measuring the lean body mass of the tibialis anterior (TA) in a dexamethasone-treated group (dexamethasone, excipient) and an oxiracetam-administered group. FIG. 8 is a graph showing the results of measuring grip strengths in a dexamethasone-treated group (dexamethasone, excipient) and an oxiracetam-administered group. Here, the oxiracetam-administered group was represented as 'Dex+Oxiracetam (30 mg/kg)' on the drawing.

FIGS. 5 and 8 show the results of confirming the effect of preventing or treating skeletal muscle atrophy or sarcopenia according to the oral administration of oxiracetam, and specifically show tibialis anterior (TA) diameters, the lean body mass of the tibialis anterior (TA), the lean body mass of the gastrocnemius muscle (GS), and grip strengths in the dexamethasone-treated group (dexamethasone, excipient) and the oxiracetam-administered group.

As shown in FIGS. 5 and 7, it can be seen that the tibialis anterior (TA) diameter in the oxiracetam-administered group was significantly increased compared to that of the dexamethasone-treated group (dexamethasone, excipient).

As shown in FIGS 6 and 7, in the case of the lean body mass of the tibialis anterior (TA) and the lean body mass of the gastrocnemius muscle (GS), it can be seen that the decrease was significantly suppressed in the oxiracetam-administered group compared to the dexamethasone-treated group (dexamethasone, excipient). That is, it can be seen that, in the dexamethasone-treated group (dexamethasone, excipient), lean body mass was significantly reduced from the time when dexamethasone was first treated, but in the oxiracetam-administered group, the decrease was not significant even though dexamethasone was treated.

As shown in FIG. 8, it can be confirmed that the oxiracetam-administered group had significantly increased grip strength compared to the dexamethasone-treated group (dexamethasone, excipient).

Taken together, it can be seen that, compared to the dexamethasone-treated group (dexamethasone, excipient), in the oxiracetam-administered group, skeletal muscle atrophy or muscle reduction is excellently inhibited. It can be seen that the same tendency is shown in aging mice.

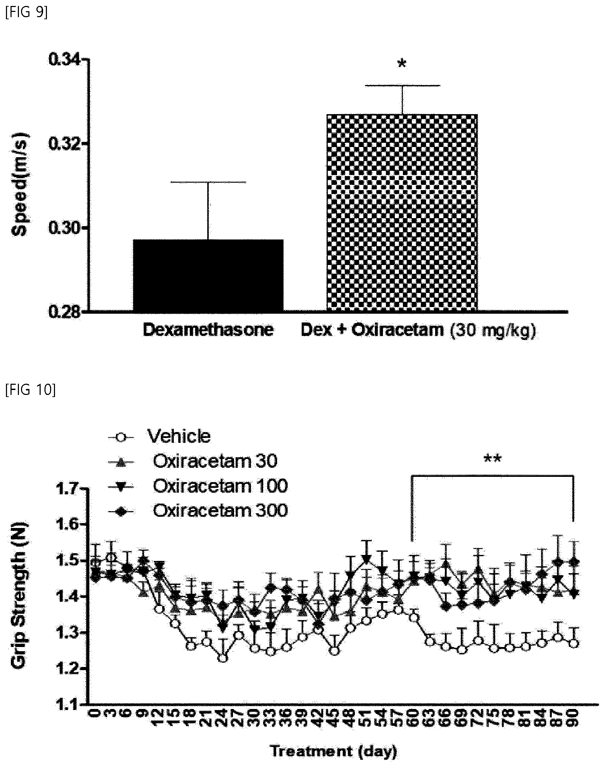

FIG. 9 is a graph showing the results of an exercise stress test in a dexamethasone-treated group (dexamethasone, excipient) and an oxiracetam-administered group, and according to this, it can be confirmed that the oxiracetam-administered group has a significant improvement in athletic ability (grip strength and endurance) compared to the dexamethasone-treated group (dexamethasone, excipient).

### Experimental Example 2. Analysis of oxiracetam efficacy in aging animal model

### 2-1. Preparation of experimental animals and samples

For an experimental group in the present invention, oxiracetam having a structure of Formula 1 was purchased from Tokyo Chemical Industry (Japan; Product No. O0398, CAS RN:62613-82-5, purity: 96%) and used.

As a model of sarcopenia caused by aging, 22-month-old male C57BL/6J mice were used. Until the day of the experiment, general feed (EEGJ30060: Cargill Agri Purina, Seongnam, Korea) and water were sufficiently supplied to the experimental animals, and the animals were acclimated for 1 week under an environment of a temperature of 23 ± 2 °C, a humidity of 55 ± 10%, and a light-dark cycle of 12 hours-12 hours and used in the experiment.

The animals were randomly divided into groups of 10 animals, such as a control (vehicle, n=10), and 30, 100, and 300 mg/kg/day oxiracetam-administered groups (n=10 per concentration). To the oxiracetam-administered group, oxiracetam was orally administered using a Zonde needle for 90 days once a day at a dose of 30, 100, or 300 mg/kg/day. During the same period, no drug was administered to the control group, and 100 µL of normal saline was orally administered at a time.

**[Table 2]**

| Classification | Description |
|---|---|
| Control (vehicle) | 22-month-old mouse + Administered normal saline daily for 90 days (vehicle) |
| Oxiracetam 30-administered group (Oxi 30) | 22-month-old mouse + Administered oxiracetam daily for 90 days (30 mg/kg/day) |
| Oxiracetam 100-administered group (Oxi 100) | 22-month-old mouse + Administered oxiracetam daily for 90 days (100 mg/kg/day) |
| Oxiracetam 300-administered group (Oxi 300) | 22-month-old mouse + Administered oxiracetam daily for 90 days (300 mg/kg/day) |

### 2-2. Grip strength measurement

To measure grip strength, a grip strength test was performed. The maximum force presented when the forelimbs of an animal model of each group could no longer hold the rod by pulling the tail horizontally while holding the tail of the animal model and holding the rod of a device with both front paws was considered to be grip strength (N). The grip strength was measured once every 3 days, and after repeating five measurements at one time, the average value was shown. Grip strength adaptation training was performed at least three times before drug administration.

### 2-3. Change in lean body mass

The lean body mass of the gastrocnemius muscle (GS) and the lean body mass of the tibialis anterior (TA) for each group was measured every 7 days. Specifically, animal models of each group were anesthetized using 3% isoflurane, fixed to a photographing bed and then photographed. During photographing, the respiratory rates and body temperatures of the animal models were measured with an animal monitoring system (SA Instruments, USA).

The whole body of the animal model was photographed through dual energy X-ray absorptiometry (DEXA) using iNSiGHT VET DXA (Osteosys, KR), and the lean body mass of each of the gastrocnemius muscle (GS) and the tibialis anterior (TA) was measured and calculated from the two-dimensional images of the gastrocnemius muscle (GS) and the tibialis anterior (TA) by a program using the region of interest (ROI).

### 2-4. Exercise stress test (treadmill test)

At 90 days after the start of the experiment, an exercise stress test was performed. First, the experimental animals in each group were acclimatized to a treadmill machine twice before measurement (1^{st} round: inclination 10°, 10 minutes at 5 m/min and 10 minutes at 10 m/min; 2^{nd} round: inclination 10°, 5 minutes at 5 m/min and 15 minutes at 10 m/min). On the day of the experiment, the experimental animals were allowed to run for 20 minutes at an initial speed of 10 m/min at an inclination of 10°, and then the speed was increased by 2 m/min every 2 minutes, and then the running distance and time were measured with the end point when the experimental animal stayed for 10 seconds without running.

### 2-5. Statistical analysis

Statistical processing was performed using GraphPad Prism (GraphPad Software Inc., San Diego, CA, USA). The results of each repeated experiment were expressed as mean ± standard error of mean, and analyzed using the analysis of variance (Tukey's test), and statistical significance was acknowledged when the P-value was 0.05 or less.

FIG. 10 is a graph showing the change in grip strength according to an administration of oxiracetam (oxiracetam 30, oxiracetam 100 and oxiracetam 300) of aging mice. The control (vehicle) was not administered a drug, and was a normal saline-treated group. In the case of the control, which is an aging (22-month-old) animal model, it can be confirmed that the grip strength is significantly reduced over time. On the other hand, it can be seen that, in the group treated with oxiracetam (oxiracetam 30, oxiracetam 100 and oxiracetam 300), the decline in grip strength is suppressed and rather increased.

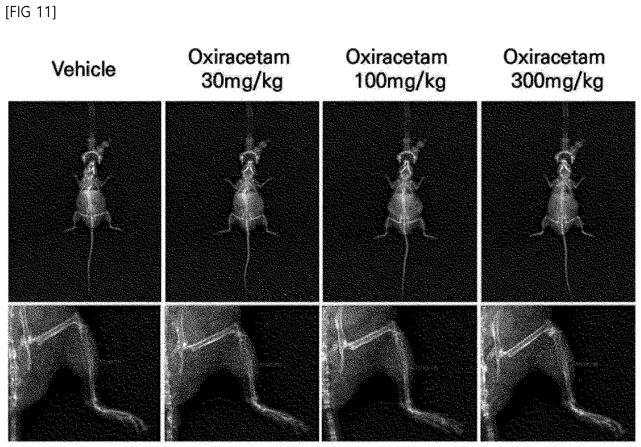

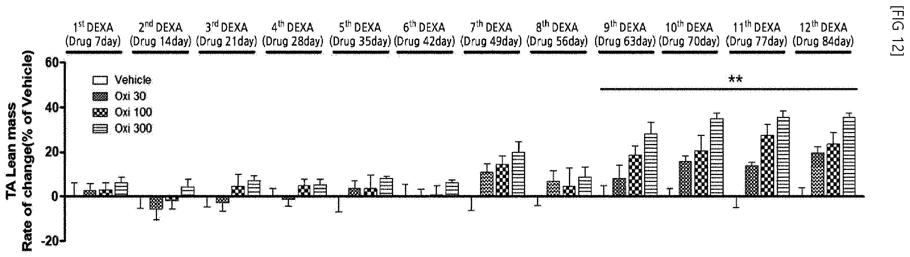

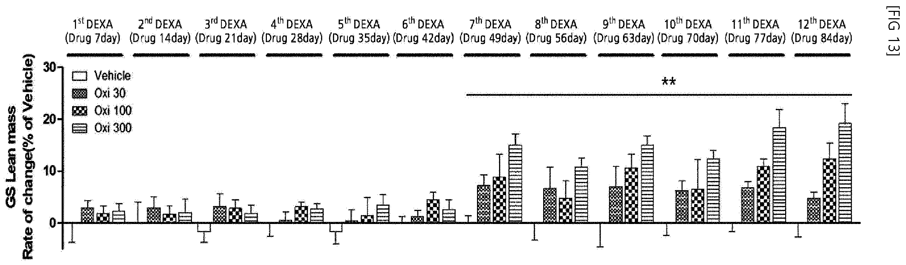

FIG. 11 is a set of representative images of a control (vehicle) and oxiracetam-administered groups (oxiracetam 30, oxiracetam 100 and oxiracetam 300) analyzed by dual energy X-ray absorptiometry (DEXA) on day 83, FIG. 12 is a graph obtained by measuring the lean body mass of the tibialis anterior (TA) every 7 days according to a control (vehicle) and oxiracetam-administered groups (oxiracetam 30, oxiracetam 100 and oxiracetam 300), and FIG. 13 is a graph obtained by measuring the lean body mass of the gastrocnemius muscle (GS) every 7 days according to a control (vehicle) and oxiracetam-administered groups (oxiracetam 30, oxiracetam 100 and oxiracetam 300).

As shown in FIGS. 12 and 13, it can be confirmed that, in the oxiracetam-treated groups (Oxi 30, Oxi 100, Oxi 300), compared to the control, which is an aging (22-month-old) animal model, the lean body mass of the tibialis anterior (TA) and the lean body mass of the gastrocnemius muscle (GS) is increased rather than decreased, and from week 7, it can be confirmed that the lean body mass is stably improved. Particularly, in the case of the Oxi 300 group, it can be confirmed that the lean body mass of the gastrocnemius muscle (GS) is significantly increased from week 7.

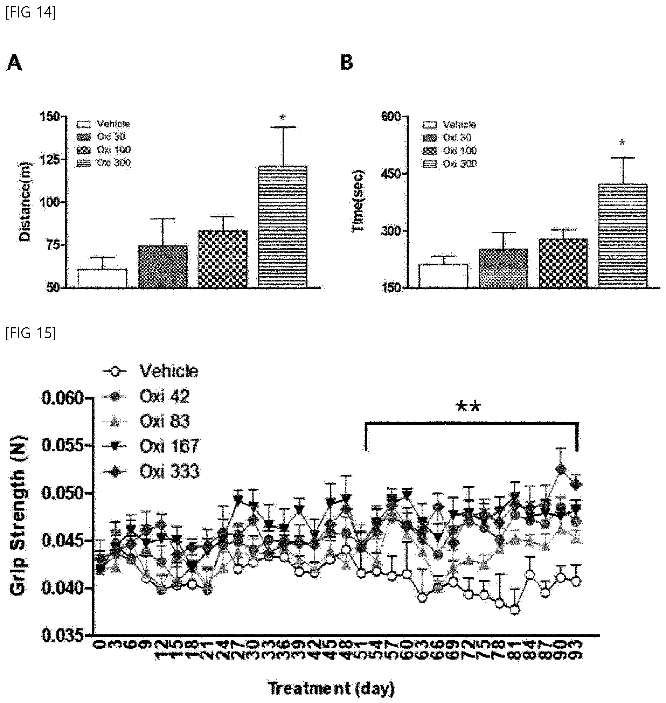

FIG. 14 is a graph showing the results of an exercise stress test for muscle function in a control (vehicle) and oxiracetam-administered groups (Oxi 30, Oxi 100, Oxi 300), and FIG. 14A is the measured running distance (m), and FIG. 14B is the measured running time (sec).

As shown in FIG. 14, it can be confirmed that, in the oxiracetam-treated groups (Oxi 30, Oxi 100, Oxi 300), compared to the control, which is an aging (22-month-old) animal model, the physical performance capacity (athletic ability) was increased rather than decreased, and in the Oxi 300 group, the physical performance capacity (athletic ability) was significantly increased.

Therefore, it can be seen that the oxiracetam according to the present invention has the effect of increasing/recovering muscle with respect to muscle reduction or muscle loss induced by aging, which is a symptom of sarcopenia, that is beyond the inhibition and prevention thereof.

### Experimental Example 3. Analysis of efficacy of oxiracetam per concentration in aging animal model

### 3-1. Preparation of experimental animals and samples

For an experimental group in the present invention, oxiracetam having a structure of Formula 1 was purchased from Tokyo Chemical Industry (Japan; Product No. O0398, CAS RN:62613-82-5, purity: 96%) and used.

As a model of sarcopenia caused by aging, 24-month-old male C57BL/6J mice were used. Until the day of the experiment, general feed (EEGJ30060: Cargill Agri Purina, Seongnam, Korea) and water were sufficiently supplied to the experimental animals, and the animals were acclimated for 1 week under an environment of a temperature of 23 ± 2 °C, a humidity of 55 ± 10%, and a light-dark cycle of 12 hours-12 hours and used in the experiment.

The animals were randomly divided into groups of 10 animals, such as a control (vehicle, n=10), and 42, 83, 167, 333 mg/kg/day oxiracetam-administered groups (n=10 per concentration). To the oxiracetam-administered group, oxiracetam was orally administered using a Zonde needle for 93 days once a day at a dose of 42, 83, 167, or 333 mg/kg/day. During the same period, no drug was administered to the control group, and 100 µL of normal saline was orally administered at one time.

**[Table 3]**

| Classification | Description |
|---|---|
| Control (vehicle) | 24-month-old mouse + Administered normal saline daily for 93 days (vehicle) |
| Oxiracetam 42-administered group (Oxi 42) | 24-month-old mouse + Administered oxiracetam daily for 93 days (42 mg/kg/day) |
| Oxiracetam 83-administered group (Oxi 83) | 24-month-old mouse + Administered oxiracetam daily for 93 days (83 mg/kg/day) |
| Oxiracetam 167-administered group (Oxi 167) | 24-month-old mouse + Administered oxiracetam daily for 93 days (167 mg/kg/day) |
| Oxiracetam 333-administered group (Oxi 333) | 24-month-old mouse + Administered oxiracetam daily for 93 days (333 mg/kg/day) |

### 3-2. Grip strength measurement

To measure grip strength, a grip strength test was performed. The maximum force presented when the forelimbs of an animal model of each group could no longer hold the rod by pulling the tail horizontally while holding the tail of the animal model and holding the rod of a device with both front paws was considered to be grip strength (N). The grip strength was measured once every 3 days, and after repeating five measurements at one time, the average value was shown. Grip strength adaptation training was performed at least three times before drug administration.

### 3-3. Change in lean body mass

The lean body mass for the gastrocnemius muscle (GS) and tibialis anterior (TA) for each group was measured every 7 days. Specifically, animal models of each group were anesthetized using 3% isoflurane, fixed to a photographing bed, and then photographed. The respiration rates and temperatures of the animal models during photographing were measured using an animal monitoring system (SA Instruments, USA).

The whole body of the animal model was photographed through dual energy X-ray absorptiometry (DEXA) using iNSiGHT VET DXA (Osteosys, KR), and the lean body mass of each of the gastrocnemius muscle (GS) and the tibialis anterior (TA) was measured and calculated from the two-dimensional images of the gastrocnemius muscle (GS) and the tibialis anterior (TA) by a program using the region of interest (ROI).

### 3-4. Statistical analysis

Statistical processing was performed using GraphPad Prism (GraphPad Software Inc., San Diego, CA, USA). The results of each repeated experiment were expressed as mean ± standard error of mean, and analyzed using the analysis of variance (Tukey's test), and statistical significance was acknowledged when the P-value was 0.05 or less.

FIG. 15 is a graph showing the results of the change in grip strength in a control (vehicle) and oxiracetam-administered groups (Oxi 42, Oxi 83, Oxi 167, Oxi 333), and in the case of the control, which is an aging (24-month-old) animal model, it can be confirmed that the grip strength has considerably declined over time. On the other hand, it can be confirmed that, in the oxiracetam-treated groups (Oxi 42, Oxi 83, Oxi 167 and Oxi 333), a decrease in grip strength is significantly inhibited and rather increased. It was confirmed that the effect of oxiracetam increases in a concentration-dependent manner.

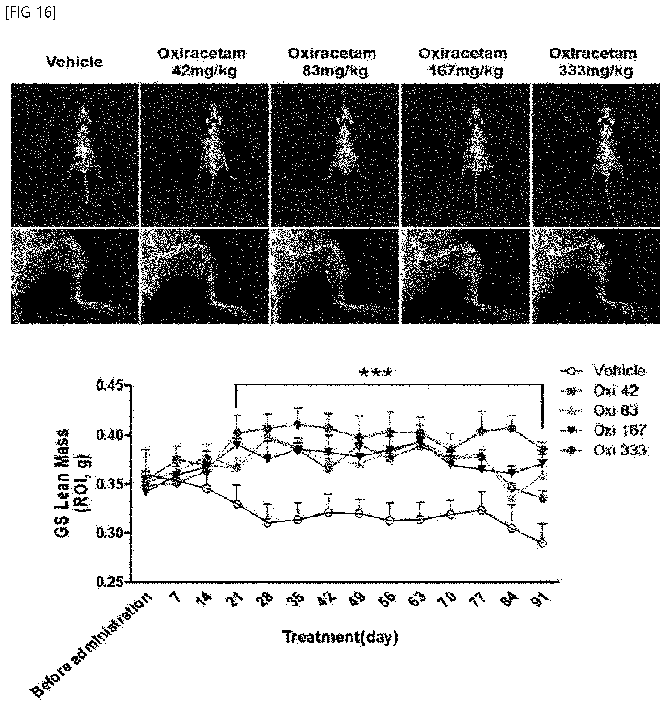

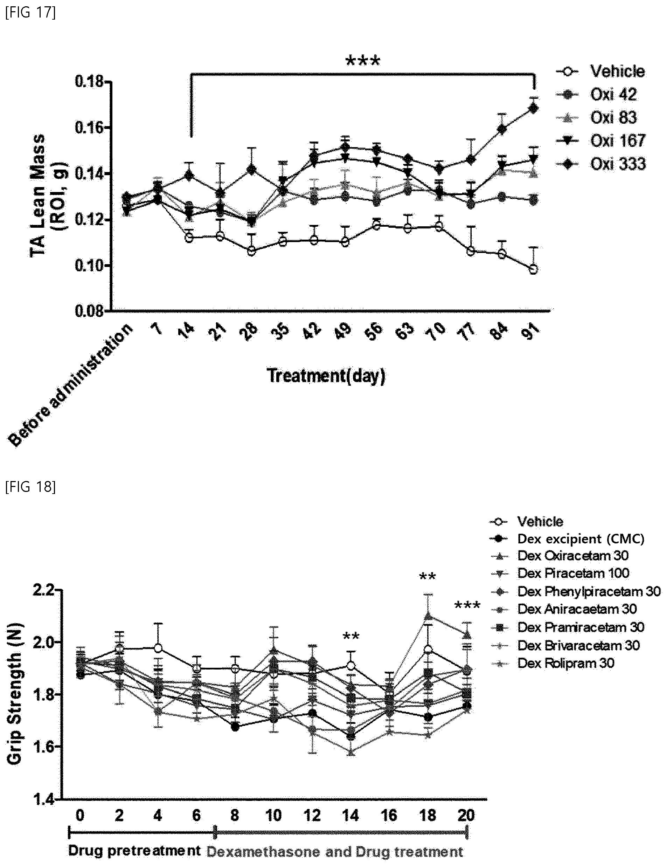

FIG. 16 is a set of representative images of a control (vehicle) and oxiracetam-administered groups (Oxi 42, Oxi 83, Oxi 167 and Oxi 333) analyzed by dual energy X-ray absorptiometry (DEXA) on day 91, and a graph showing the results of measuring the lean body mass of the gastrocnemius muscle (GS) every 7 days, and FIG. 17 is a graph showing the results of measuring the lean body mass of the tibialis anterior (TA) every 7 days in a control (vehicle) and oxiracetam-administered groups (Oxi 42, Oxi 83, Oxi 167 and Oxi 333).

As shown in FIGS. 16 and 17, it can be confirmed that, in a control, which is an aging (24-month-old) animal model, the lean body mass of the gastrocnemius muscle (GS) and the lean body mass of the tibialis anterior (TA) are significantly and greatly decreased over time. On the other hand, in the oxiracetam-treated groups (Oxi 42, Oxi 83, Oxi 167 and Oxi 333), compared to the control, which is an aging (24-month-old) animal model, it was confirmed that the lean body mass of the gastrocnemius muscle (GS) and the lean body mass of the tibialis anterior (TA) are rather significantly increased, and the Oxi 333 group shows the greatest increase.

Therefore, it can be seen that the oxiracetam according to the present invention has an effect of increasing/recovering muscle with respect to muscle reduction or muscle loss induced by aging, which is a symptom of sarcopenia, that is beyond the inhibition and prevention thereof.

### Experimental Example 4. Comparison in efficacy with racetam-based drug

### 4-1. Preparation of experimental animals and samples

For an experimental group in the present invention, oxiracetam having a structure of Formula 1 was purchased from Tokyo Chemical Industry (Japan; Product No. O0398, CAS RN:62613-82-5, purity: 96%) and used. Other racetam drugs, such as aniracetam and nepiracetam, were purchased from Bide Pharmatech ( 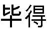 ; China, Product Nos. BD114391, BD140804, purity: 96%), piracetam, phenylpiracetam, corulacetam and pramiracetam were purchased from Tokyo Chemical Industry (Japan, Product Nos. P2880, P2604, C3689, P2061, purity: 96%), levetiracetam was purchased from Roeun ( 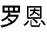 ; China, Product No. R025860, purity: 96%), brivaracetam was purchased from R&D Systems (USA, Product No. 7271, purity: 96%), rolziracetam and faroracetam were purchased from Targetmol (USA, Product Nos. T16783, T19624, purity: 96%), and rolipram was purchased from Aladdin ( 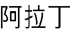 ; China, Product No. R129674, purity: 96%).

As experimental animals, 7-week-old male ICR mice were purchased and used. Until the day of the experiment, general feed (EEGJ30060: Cargill Agri Purina, Seongnam, Korea) and water were sufficiently supplied to the experimental animals, and the animals were acclimated for 1 week under an environment of a temperature of 23 ± 2 °C, a humidity of 55 ± 10%, and a light-dark cycle of 12 hours-12 hours and used in the experiment.

After acclimation of the experimental animals, to create a muscular atrophy model, the mice were randomly divided into groups of 7 animals, such as a control (vehicle, n=7), a dexamethasone-treated group (n=7), and a drug-administered group (n=7). To the groups except for the control, dexamethasone was intraperitoneally injected at a dose of 10 mg/kg from day 7 to day 21 for 14 days. To the drug-administered group, a drug was orally administered at a dose of 30 or 100 mg/kg/day once a day for 21 days using a Zonde needle from 7 days before intraperitoneal administration of dexamethasone. During the same period, no drug was administered to the control, and 100 µL of normal saline was orally administered at a time. To the dexamethasone-treated group (dexamethasone, excipient), instead of the drug, 100 µL of a 0.5% carboxymethylcellulose (CMC) solution was administered. This is to set the effect on the excipient as a blank.

**[Table 4]**

| Classification | Description |
|---|---|
| Control (vehicle) | Administered normal saline daily for 21 days (vehicle) |
| Dexamethasone-treated group (Dexamethasone, excipient; CMC) | Administered dexamethasone daily from day 7 to day 21 for 14 days (10 mg/kg/day) + Administered excipient (CMC) daily from day 1 to day 21 |
| Oxiracetam 30-administered group (Oxiracetam 30) | Administered dexamethasone daily from day 7 to day 21 for 14 days (10 mg/kg/day) + Administered oxiracetam daily from day 1 to day 21 (30 mg/kg/day) |
| Piracetam 100-administered group (Piracetam 100) | Administered dexamethasone daily from day 7 to day 21 for 14 days (10 mg/kg/day) + Administered piracetam daily from day 1 to day 21 (100 mg/kg/day) |
| Phenylpiracetam 30-administered group (Phenylpiracetam 30) | Administered dexamethasone daily from day 7 to day 21 for 14 days (10 mg/kg/day) + Administered phenylpiracetam daily from day 1 to day 21 (30 mg/kg/day) |
| Aniracetam 30-administered group (Aniracetam 30) | Administered dexamethasone daily from day 7 to day 21 for 14 days (10 mg/kg/day) + Administered aniracetam daily from day 1 to day 21 (30 mg/kg/day) |
| Levetiracetam 30-administered group (Levetiracetam 30) | Administered dexamethasone daily from day 7 to day 21 for 14 days (10 mg/kg/day) + Administered levetiracetam daily from day 1 to day 21 (30 mg/kg/day) |
| Nefiracetam 30-administered group (Nefiracetam 30) | Administered dexamethasone daily from day 7 to day 21 for 14 days (10 mg/kg/day) + Administered nefiracetam daily from day 1 to day 21 (30 mg/kg/day) |
| Coluracetam 30-administered group (Coluracetam 30) | Administered dexamethasone daily from day 7 to day 21 for 14 days (10 mg/kg/day) + Administered coluracetam daily from day 1 to day 21 (30 mg/kg/day) |
| Rolziracetam 30-administered group (Rolziracetam 30) | Administered dexamethasone daily from day 7 to day 21 for 14 days (10 mg/kg/day) + Administered rolziracetam daily from day 1 to day 21 (30 mg/kg/day) |
| Fasoracetam 30-administered group (Fasoracetam 30) | Administered dexamethasone daily from day 7 to day 21 for 14 days (10mg/kg/day) + Administered fasoracetam daily from day 1 to day 21 (30mg/kg/day) |
| Pramiracetam 30-administered group (Pramiracetam 30) | Administered dexamethasone daily from day 7 to day 21 for 14 days (10 mg/kg/day) + Administered pramiracetam daily from day 1 to day 21 (30 mg/kg/day) |
| Brivaracetam 30-administered group (Brivaracetam 30) | Administered dexamethasone daily from day 7 to day 21 for 14 days (10 mg/kg/day) + Administered brivaracetam daily from day 1 to day 21 (30 mg/kg/day) |
| Rolipram 30-administered group (Rolipram 30) | Administered dexamethasone daily from day 7 to day 21 for 14 days (10 mg/kg/day) + Administered rolipram daily from day 1 to day 21 (30 mg/kg/day) |

### 4-2. Grip strength measurement

To measure grip strength, a grip strength test was performed. The maximum force presented when the forelimbs of an animal model of each group could no longer hold the rod by pulling the tail horizontally while holding the tail of the animal model and holding the rod of a device with both front paws was considered to be grip strength (N). Grip strength adaptation training was performed at least three times before drug administration. After measurement before drug administration (day 0) and on day 20 after drug administration began, grip strength variation (%) after drug administration began (day 20) with respect to that before drug administration (day 0) was calculated.

### 4-3. Change in lean body mass

The lean body mass of the gastrocnemius muscle (GS) and the lean body mass of the tibialis anterior (TA) for each group were measured every 7 days. Specifically, animal models of each group were anesthetized using 3% isoflurane, fixed to a photographing bed and then photographed. During photographing, the respiratory rates and body temperatures of the animal models were measured with an animal monitoring system (SA Instruments, USA).

The whole body of the animal model was photographed through dual energy X-ray absorptiometry (DEXA) using iNSiGHT VET DXA (Osteosys, KR), and the lean body mass of each of the gastrocnemius muscle (GS) and the tibialis anterior (TA) was measured and calculated from the two-dimensional images of the gastrocnemius muscle (GS) and the tibialis anterior (TA) by a program using the region of interest (ROI).

### 4-4. Statistical analysis

Statistical processing was performed using GraphPad Prism (GraphPad Software Inc., San Diego, CA, USA). The results of each repeated experiment were expressed as mean ± standard error of mean, and analyzed using the analysis of variance (Tukey's test), and statistical significance was acknowledged when the P-value was 0.05 or less.

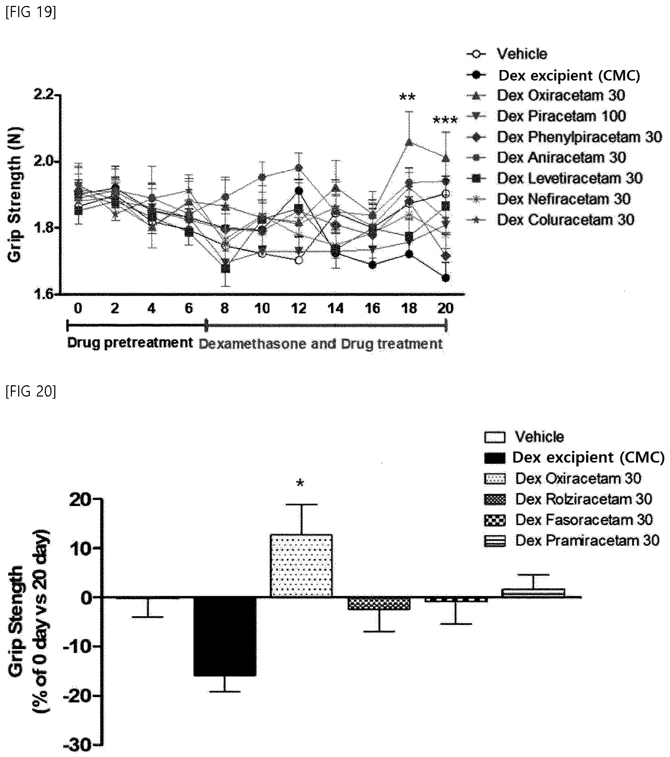

FIG. 18 is a graph showing the results of measuring the change in grip strength in a control (vehicle), a dexamethasone-treated group (dexamethasone, excipient), and drug-administered groups (oxi 30, pi 100, phenylpi 30, ani 30, prami 30, briva 30, roli 30), FIG. 19 is a graph showing the results of measuring the change in grip strength in a control (vehicle), a dexamethasone-treated group (dexamethasone, excipient), and drug-administered groups (oxi 30, pi 100, phenylpi 30, ani 30, leveti 30, nefi 30 and colu 30), and FIG. 20 is a graph showing the results of measuring the change in grip strength in a control (vehicle), a dexamethasone-treated group (dexamethasone, excipient), and drug-administered groups (oxi 30, rolzi 30, faso 30 and prami 30).

As shown in FIGS. 18, 19 and 20, the control showed increased grip strength or no change in grip strength. However, in the dexamethasone-treated group, a significant decrease in grip strength over time can be confirmed. Here, as a result of treating racetam-based drugs having the same mechanism as oxiracetam as a comparative group, it can be seen that, except for oxiracetam, there was no significant effect of recovering grip strength.

In the oxiracetam-administered group (Oxi 30), through administration of 30 mg/kg/day, it can be confirmed that the grip strength of an animal model in which muscular atrophy was induced by dexamethasone is not only prevented from decreasing but also recovers to greater than the control. Therefore, it can be confirmed that, although a racetam-based drug has the same mechanism as oxiracetam, it does not have the effect of preventing or treating sarcopenia like the oxiracetam of the pharmaceutical composition for use according to the present invention.

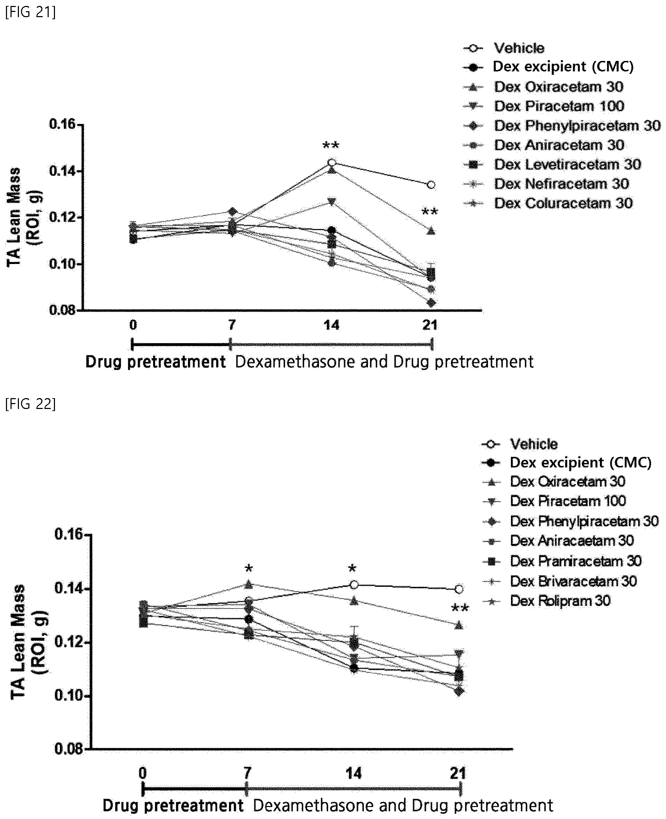

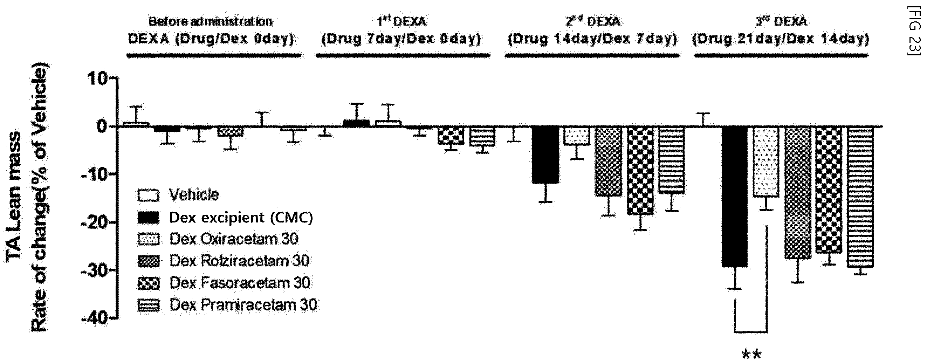

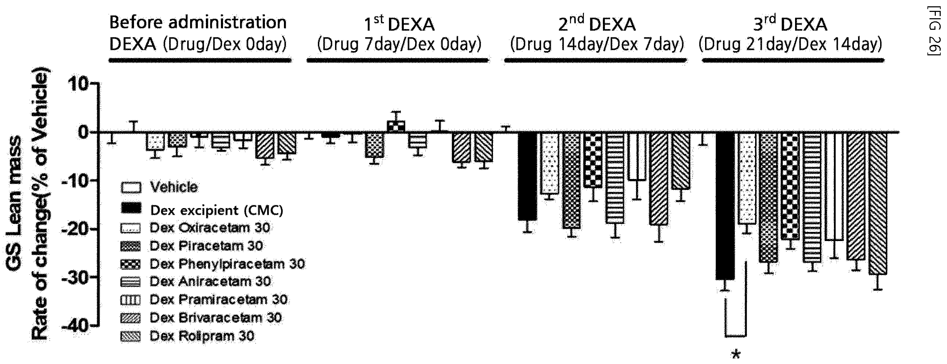

FIG. 21 is a graph showing the results of measuring the lean body mass of tibialis anterior (TA) every 7 days in a control (vehicle), a dexamethasone-treated group (dexamethasone, excipient), and drug-administered groups (oxi 30, pi 100, phenylpi 30, ani 30, leveti 30, nefi 30 and colu 30), FIG. 22 is a graph showing the results of measuring the lean body mass of the tibialis anterior (TA) every 7 days in a control (vehicle), a dexamethasone-treated group (dexamethasone, excipient), and drug-administered groups (oxi 30, pi 100, phenylpi 30, ani 30, prami 30, briva 30 and roli 30), FIG. 23 is a graph showing the results of measuring the lean body mass of the tibialis anterior (TA) every 7 days in a control (vehicle), a dexamethasone-treated group (dexamethasone, excipient), and drug-administered groups (oxi 30, rolzi 30, faso 30 and prami 30), and FIG. 26 shows the variation in lean body mass (%) of the drug-administered group, calculated with respect to the control (vehicle).

As shown in FIGS. 21, 22 and 23, it can be confirmed that, in the control, there is no change in lean body mass of the tibialis anterior (TA), but in the dexamethasone-treated group, the lean body mass of the tibialis anterior (TA) has significantly decreased over time. Here, as a result of treating racetam-based drugs having the same mechanism as oxiracetam as a control, by all of the racetam-based drugs except for oxiracetam, the lean body mass of the tibialis anterior (TA) is not recovered but rather reduced like in the dexamethasone-treated group.

On the other hand, it can be confirmed that, in the oxiracetam-administered group (Oxi 30), the administration of 30 mg/kg/day cannot only prevent the decrease in lean body mass of the tibialis anterior (TA) of the animal model in which muscular atrophy is induced by dexamethasone, but also recover the lean body mass of the tibialis anterior (TA) to the control (vehicle) level. Therefore, it can be confirmed that, although a racetam-based drug has the same mechanism as oxiracetam, it does not have the effect of preventing or treating sarcopenia like the oxiracetam in the pharmaceutical composition for use according to the present invention.

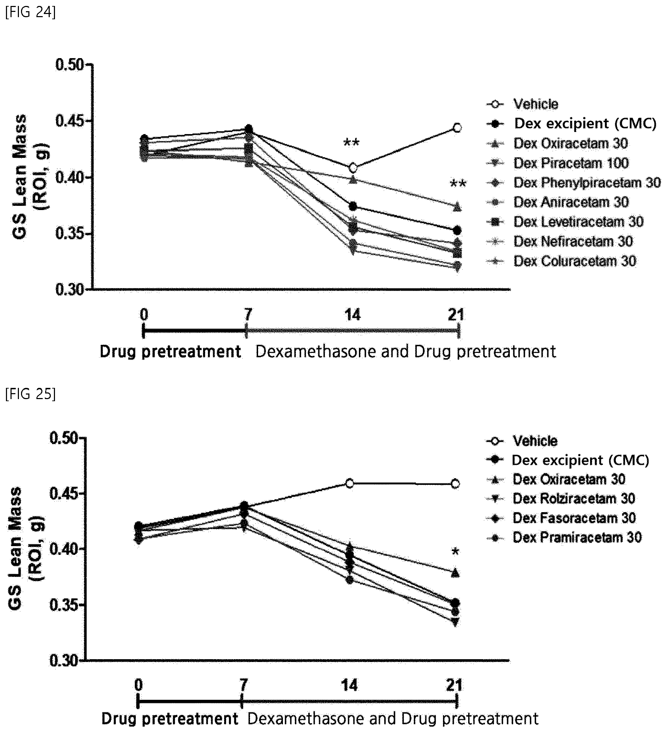

FIG. 24 is a graph showing the results of measuring the lean body mass of the gastrocnemius muscle (GS) every 7 days in a control (vehicle), a dexamethasone-treated group (dexamethasone, excipient), and drug-administered groups (oxi 30, pi 100, phenylpi 30, ani 30, leveti 30, nefi 30 and colu 30), FIG. 25 is a graph showing the results of measuring the lean body mass of the gastrocnemius muscle (GS) every 7 days in a control (vehicle), a dexamethasone-treated group (dexamethasone, excipient), and drug-administered groups (oxi 30, rolzi 30, faso 30 and prami 30), and FIG. 26 is a graph showing the results of measuring the lean body mass of the gastrocnemius muscle (GS) every 7 days in a control (vehicle), a dexamethasone-treated group (dexamethasone, excipient), and drug-administered groups (oxi 30, pi 100, phenylpi 30, ani 30, prami 30, briva 30 and roli 30).

As shown in FIGS. 24, 25 and 26, it can be confirmed that, in the control, there is no change in lean body mass, but in the dexamethasone-treated group, the lean body mass of the gastrocnemius muscle (GS) is significantly decreased over time. Here, as a result of treating racetam-based drugs having the same mechanism as oxiracetam as comparative groups, it can be seen that all of the racetam-based drugs except for oxiracetam cannot recover the lean body mass of the gastrocnemius muscle (GS) and significantly reduce the lean body mass of the gastrocnemius muscle (GS) like the dexamethasone-treated group (dexamethasone, excipient).

On the other hand, it can be confirmed that, in the oxiracetam-administered group (Oxi 30), the administration of 30 mg/kg/day cannot only prevent the decrease in lean body mass of the gastrocnemius muscle (GS) of the animal model in which muscular atrophy is induced by dexamethasone, but also recover the lean body mass of the gastrocnemius muscle (GS) to the control (vehicle) level. Therefore, it can be confirmed that, although a racetam-based drug has the same mechanism as oxiracetam, it cannot have the effect of preventing or treating sarcopenia like the oxiracetam in the pharmaceutical composition for use according to the present invention.

Through the above-described experiment, compared to other racetam-based drugs (piracetam, oxiracetam, phenylpiracetam, aniracetam, levetiracetam, nefiracetam, coluracetam, rolziracetam, fasoracetam, pramiracetam, brivaracetam, and rolipram), it can be confirmed that oxiracetam, which is the active ingredient of the pharmaceutical composition for use according to the present invention, has the significant efficacy of recovering grip strength and lean body mass. Particularly, it can be confirmed that oxiracetam is a piracetam derivative but has the significant effect of recovering grip strength and muscle mass compared to piracetam.

### Experimental Example 5. Comparison of efficacy with dementia drug

### 5-1. Preparation of experimental animals and samples

For an experimental group in the present invention, oxiracetam having a structure of Formula 1 was purchased from Tokyo Chemical Industry (Japan; Product No. O0398, CAS RN:62613-82-5, purity: 96%) and used. Mirtazapine and olanzapine were purchased from Tokyo Chemical Industry (Japan, Product Nos. M2151, O0393, purity: 96%) and used.

As experimental animals, 7-week-old male ICR mice were purchased and used. Until the day of the experiment, general feed (EEGJ30060: Cargill Agri Purina, Seongnam, Korea) and water were sufficiently supplied to the experimental animals, and the animals were acclimated for 1 week under an environment of a temperature of 23 ± 2 °C, a humidity of 55 ± 10%, and a light-dark cycle of 12 hours-12 hours and used in the experiment.

After the acclimation period for the experimental animals, to create a muscular atrophy model, the mice were randomly divided into groups of 7 animals, such as a control (vehicle, n=7), a dexamethasone-treated group (n=7), and a drug-administered group (n=7). The groups except for the control were intraperitoneally injected dexamethasone at a dose of 10 mg/kg from day 7 to day 21 for 14 days. In the case of the drug-administered group, 7 days before the intraperitoneal administration of dexamethasone, the drug was orally administered using a Zonde needle at a dose of 30 or 10 mg/kg/day once a day for 21 days. During the same period, the control was administered no drug, and 100 µL of normal saline was orally administered at a time. To the dexamethasone-treated group (dexamethasone excipient, CMC), instead of the drug, 100 µL of a 0.5% carboxymethylcellulose (CMC) solution was administered. This is to set the effect on the excipient as a blank.

**[Table 5]**

| Classification | Description |
|---|---|
| Control (vehicle) | Administered normal saline daily for 21 days (vehicle) |
| Dexamethasone-treated group (Dexamethasone, excipient; CMC) | Administered dexamethasone daily from day 7 to day 21 for 14 days (10 mg/kg/day) + Administered excipient (CMC) daily from day 1 to day 21 |
| Oxiracetam 30-administered group (Oxi 30) | Administered dexamethasone daily from day 7 to day 21 for 14 days (10 mg/kg/day) + Administered oxiracetam daily from day 1 to day 21 (30 mg/kg/day) |
| Mirtazapine 10-administered group (Mirtazapine 10) | Administered dexamethasone daily from day 7 to day 21 for 14 days (10 mg/kg/day) + Administered mirtazapine daily from day 1 to day 21 (10 mg/kg/day) |
| Olanzapine 10-administered group (Olanzapine 10) | Administered dexamethasone daily from day 7 to day 21 for 14 days (10 mg/kg/day) + Administered olanzapine daily from day 1 to day 21 (10 mg/kg/day) |

### 5-2. Grip strength measurement

To measure grip strength, a grip strength test was performed. The maximum force presented when the forelimbs of an animal model of each group could no longer hold the rod by pulling the tail horizontally while holding the tail of the animal model and holding the rod of a device with both front paws was considered to be grip strength (N). Grip strength adaptation training was performed at least three times before drug administration. After measurement before drug administration (day 0) and on day 20 after drug administration began, grip strength variation (%) after drug administration began (day 20) with respect to that before drug administration (day 0) was calculated.

### 5-3. Change in lean body mass

The lean body mass of the gastrocnemius muscle (GS) and the lean body mass of the tibialis anterior (TA) for each group were measured every 7 days. Specifically, animal models of each group were anesthetized using 3% isoflurane, fixed to a photographing bed and then photographed. During photographing, the respiratory rates and body temperatures of the animal models were measured with an animal monitoring system (SA Instruments, USA).

The whole body of the animal model was photographed through dual energy X-ray absorptiometry (DEXA) using iNSiGHT VET DXA (Osteosys, KR), and the lean body mass of each of the gastrocnemius muscle (GS) and the tibialis anterior (TA) was measured and calculated from the two-dimensional images of the gastrocnemius muscle (GS) and the tibialis anterior (TA) by a program using the region of interest (ROI).

### 5-4. Statistical analysis

Statistical processing was performed using GraphPad Prism (GraphPad Software Inc., San Diego, CA, USA). The results of each repeated experiment were expressed as mean ± standard error of mean, and analyzed using the analysis of variance (Tukey's test), and statistical significance was acknowledged when the P-value was 0.05 or less.

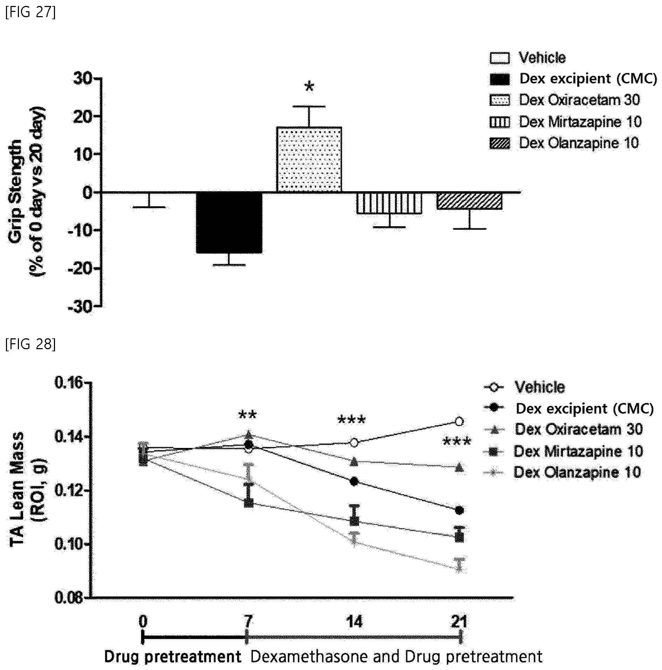

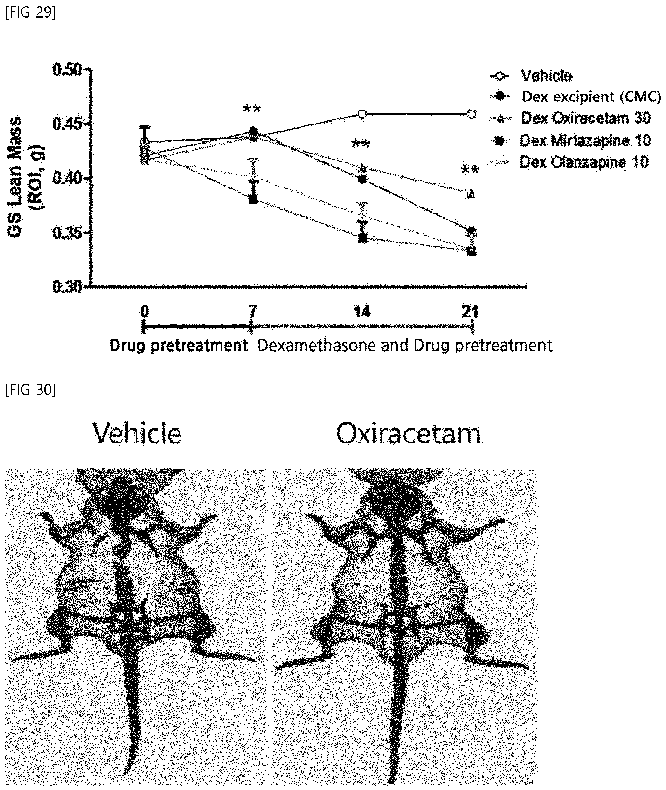

FIG. 27 is a graph showing the results of measuring the change in grip strength in a control (vehicle), a dexamethasone-treated group (dexamethasone, excipient), and drug-administered groups (Oxiracetam30, Mirtazapine10 and Olanzapine10), FIG. 28 is a graph obtained by measuring the lean body mass of the tibialis anterior (TA) every 7 days in a control (vehicle) and oxiracetam-administered groups (Oxiracetam 30, Mirtazapine 10 and Olanzapine 10), and FIG. 29 is a graph obtained by measuring the lean body mass of the gastrocnemius muscle (GS) every 7 days in a control (vehicle) and oxiracetam-administered groups (Oxiracetam 30, Mirtazapine 10 and Olanzapine 10). Here, on the graph, the dexamethasone-treated group was denoted as DEX excipient (CMC).

As shown in FIGS. 27 to 29, it can be confirmed that, in the control, there is no change in grip strength, and lean body mass of the tibialis anterior (TA) and gastrocnemius muscle (GS), but in the dexamethasone-treated group, the grip strength and the lean body mass are significantly reduced over time. As a result of treating dementia drugs such as mirtazapine and olanzapine as comparative groups, like the dexamethasone-treated group (dexamethasone, excipient), in all cases, it can be seen that the grip strength is reduced, the lean body mass of the tibialis anterior (TA) and the lean body mass of the gastrocnemius muscle (GS) are not recovered but rather reduced.

On the other hand, it can be confirmed that, in the oxiracetam-administered group (Oxi 30), the administration of 30 mg/kg/day cannot only prevent the decreases in grip strength and lean body mass of the tibialis anterior (TA) and the gastrocnemius muscle (GS) of the animal model in which muscular atrophy is induced by dexamethasone, but also significantly recovers the grip strength and the lean body mass, compared to the control (vehicle). Therefore, it can be confirmed that, although a dementia drug has the same mechanism as oxiracetam, it does not have the effect of preventing or treating sarcopenia like the oxiracetam of the pharmaceutical composition for use according to the present invention.

### Experimental Example 6. Analysis of oxiracetam efficacy in Duchenne animal model

### 6-1. Animal breeding

As experimental animals, 23-week-old male mice (C57BL/10 ScSn-Dmdmdx/J) were purchased from the Jackson Laboratory (USA) to conduct experiments. All animals were maintained under conditions of a temperature of 23 ± 2 °C and a relative humidity of 55 ± 10%. Before the initiation of the experiment, a total of 20 mice were randomly divided into groups of 10 animals. After adaptation for 1 week, 30 mg/kg of oxiracetam of Example 1 was orally administered daily for 35 days (drug-administered group, oxiracetam). Here, for the control (vehicle), instead of a sample, normal saline was orally administered.

### 6-2. Statistics

For statistical analysis of the experiment, significant differences in mean values between groups were confirmed using a paired t-test, and p < 0.05 is denoted as *, p < 0.01 is denoted as **, p < 0.001 is denoted as ***, and no significant difference is denoted as N.S. An error bar represents S.E.M.

### 6-3. Confirmation of oxiracetam effect of improving lean body mass and reducing fat

Total lean body mass, body fat content (fat, %), the lean body mass of the gastrocnemius muscle (GS), and the lean body mass of the tibialis anterior (TA) were analyzed by dual energy X-ray absorptiometry (DEXA) every 7 days from the first day (day 0) of oral administration. Specifically, animal models of each group were anesthetized using 3% isoflurane, fixed to a photographing bed and then photographed. During photographing, the respiratory rates and body temperatures of the animal models were measured with an animal monitoring system (SA Instruments, USA).

The whole body of the animal model was photographed through dual energy X-ray absorptiometry (DEXA) using iNSiGHT VET DXA (Osteosys, KR), and the total lean body mass and the body fat content (fat, %) were analyzed. In addition, from the photographed images, the two-dimensional images of the gastrocnemius muscle (GS) and the tibialis anterior (TA) were assigned to the region of interest (ROI) using a program, and the lean body mass of each of the gastrocnemius muscle (GS) and the tibialis anterior (TA) was measured and calculated. Since the DEXA image can be used to measure and analyze the lean body mass, fat, and bone density of a mouse, it is possible to measure and calculate not only detailed lean body mass but also total lean body mass and body fat content (fat, %).

### 6-4. Confirmation of oxiracetam effect of improving muscle function

Grip strength was measured every 2 days from the first day (day 0) of oral administration. The measurement of the grip strength was performed using a grip strength meter for mice (BIOSEB), a mouse was placed on a wire mesh attached to a gauge capable of monitoring the strength, and the tail of the mouse was pulled downward to measure a force of grabbing the wire mesh. The average value was recorded by repeating measurement five consecutive times.

### 6-5. Confirmation of oxiracetam effect of maintaining body weight

Body weights were measured every day from the first day (day 0) of oral administration.

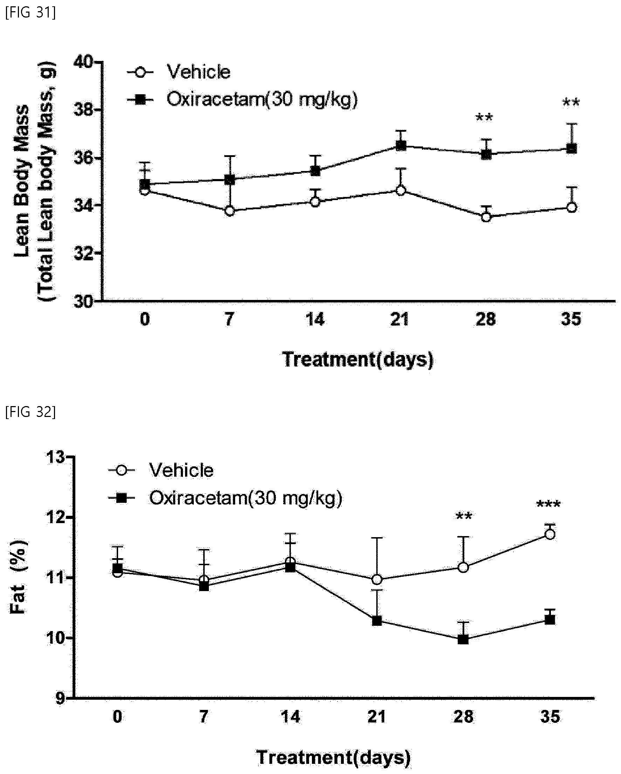

FIG. 30 is a set of images of a drug-administered group (oxiracetam) and a control (vehicle), captured by dual energy X-ray absorptiometry (DEXA), FIG. 31 is a graph showing the measurement of total lean body mass in an experimental group (oxiracetam) and a control (vehicle), and FIG. 32 is a graph showing the results of measuring a body fat mass (fat, %) in a drug-administered group (oxiracetam) and a control (vehicle).

As shown in FIGS. 30 to 32, as a result of administering oxiracetam to the DMD animal model, it can be confirmed that the total lean body mass increases and the body fat decreases. Particularly, it was confirmed that, in the drug-administered group, compared to the control, the increase in lean body mass was significantly increased on day 35, and the body fat was significantly reduced.

In addition, in the control, it can be confirmed that, according to the progression of DMD, overgrowth of connective tissue and fat accumulation occur along with fibroatrophy, and thus pseudohypertrophy, which is a characteristic symptom of DMD, is observed. On the other hand, in the case of the oxiracetam-administered group, it can be confirmed that pseudohypertrophy, which is a characteristic symptom of DMD, is reduced by suppressing muscle loss and fat accumulation.

Accordingly, it can be seen that oxiracetam has the effect of reducing pseudohypertrophy of total lean body mass in DMD caused by genetic factors.

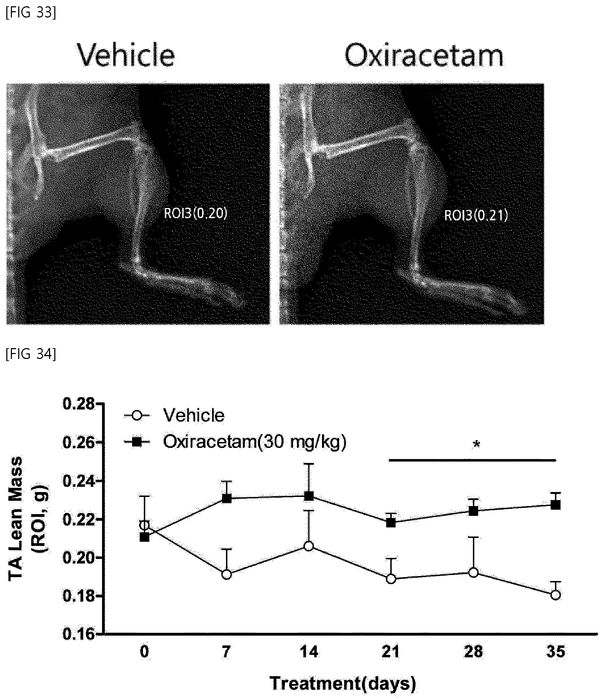

FIG. 33 is a set of images of the drug-administered group (oxiracetam) and the control (vehicle) captured by dual energy X-ray absorptiometry (DEXA), and FIG. 34 is a graph showing the results of measuring the total lean body mass of the tibialis anterior (TA) in the drug-administered group (oxiracetam) and the control (vehicle).

As shown in FIGS. 33 and 34, in the control, a characteristic symptom ofDMD, which is the dramatic decrease in lean body mass of the tibialis anterior (TA), can be confirmed. On the other hand, it can be seen that, in the oxiracetam-administered group, the lean body mass of the tibialis anterior (TA) is significantly increased, compared to the control.

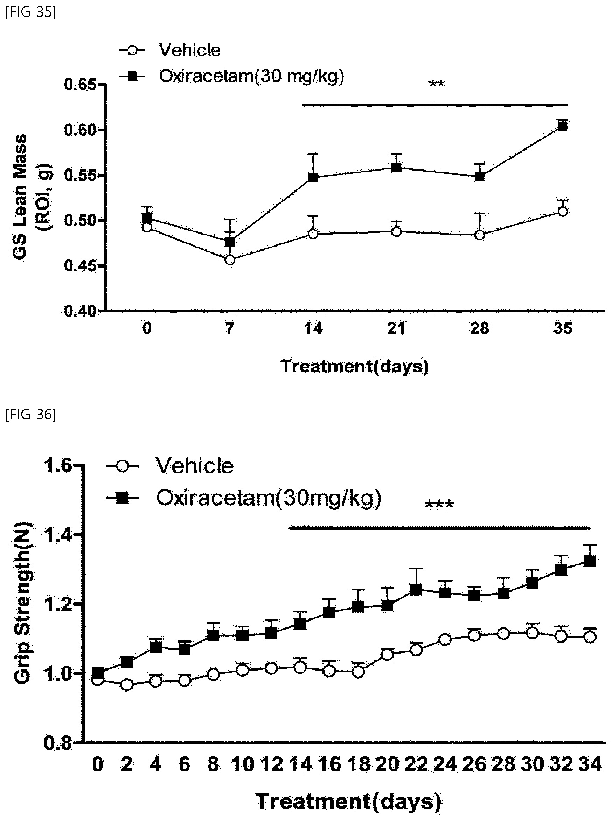

FIG. 35 is a graph showing the results of measuring the lean body mass of the gastrocnemius muscle (GS) in a drug-administered group (oxiracetam) and a control (vehicle), and accordingly, in the control, it can be seen that the decrease in lean body mass of the gastrocnemius muscle (GS), which is a characteristic symptom of DMD, gradually progresses in the control.

On the other hand, in the drug-administered group, compared to the control, it can be seen that the lean body mass of the gastrocnemius muscle (GS) was significantly increased, and thus oxiracetam significantly inhibits the decrease in lean body mass of the gastrocnemius muscle (GS) due to DMD.

FIG. 36 is a graph showing the results of measuring the change in grip strength in the drug-administered group (oxiracetam) and the control (vehicle).

As shown in FIG. 36, in the control, characteristic symptoms of DMD, which are the decline in grip strength and muscle function, were observed. On the other hand, in the drug-administered group, it was confirmed that the grip strength, which is a criterion for muscle function, is significantly increased by the oxiracetam administration. That is, it can be seen that oxiracetam exhibits a therapeutic effect on DMD.

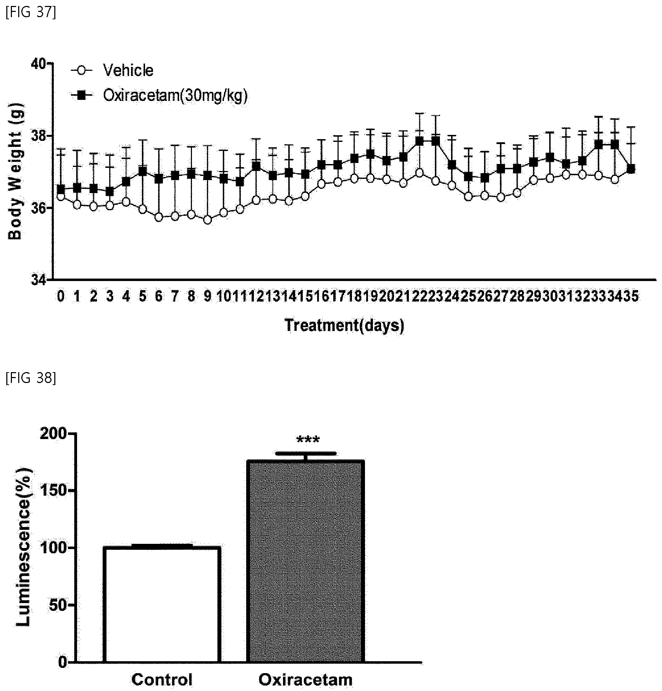

FIG. 37 is a graph showing the results of measuring the change in body weight in the drug-administered group (oxiracetam) and the control (vehicle), and when oxiracetam was administered to a model with DMD, it was confirmed that it does not exhibit a significant effect on body weight. That is, it can be seen that oxiracetam does not exhibit side effects on an animal with DMD.

### Experimental Example 7. Cell experiment for muscular atrophy

A mouse-derived C2C12 cell line was provided from the American Type Culture Collection (ATCC, CRL-1772; Manassas, VA, USA) and cultured at 37 °C in 5% CO₂. In the proliferation phase of the cells before differentiation, high concentration glucose Dulbecco's modified Eagle's medium (DMEM) containing 1% penicillin/streptomycin and 10% fetal bovine serum (FBS) medium were used, and in the differentiation induction, the FBS medium was replaced with a 2% horse serum (HS)-containing medium. The C2C12 myoblasts were seeded into a 96 well culture plate and a 6 well culture plate at 2×10⁵/well and 5×10³/well, respectively, and cultured at 37 °C in a CO₂ incubator. When the confluency reached approximately 90%, the medium was replaced with 2% horse serum (HS)-containing DMEM to induce differentiation of myoblasts. After 5 days, the C2C12 myotubes that had differentiated were treated with 10 ng/ml TNF-α, which is an inflammatory factor, for 48 hours to cause myotube atrophy, thereby creating a muscular atrophy cell model, TNF-α-treated group (TNF-α).

During the TNF-α treatment, 10 µM oxiracetam was treated to create an oxiracetam-treated group (Oxiracetam 10 µM), cells that were not treated during the same period were prepared as a control.

Two days after TNF-α and oxiracetam treatment, each of the TNF-α-treated group (TNF-α), the oxiracetam-treated group (Oxiracetam 10 µM) and the control was treated with an ATP detection reagent at room temperature, and then luminescence was measured using a mitochondrial ToxGloTM assay kit (Promega). In addition, the TNF-α-treated group (TNF-α), the oxiracetam-treated group (Oxiracetam 10 µM) and the control were photographed using an optical microscope and the diameter of a myotube cell in each group were measured and compared.

Statistical processing was performed using GraphPad Prism (GraphPad Software Inc., San Diego, CA, USA). The results of each repeated experiment were expressed as mean ± standard error of mean, and analyzed using the analysis of variance (Tukey's test), and statistical significance was acknowledged when the P-value was 0.05 or less.

FIG. 38 is a graph shown by confirming the degree of the change in ATP content in the oxiracetam-treated group (oxiracetam, 10 µM) and the control (solvent-treated, control) in myotube, and referring to this, in the oxiracetam-treated group (oxiracetam 10 µM), it can be confirmed that the ATP production activity significantly increased 50 to 80% or more than that of the control. That is, it can be seen that oxiracetam significantly enhances mitochondrial functional activity. Mitochondria are the essential cell organelle that produces cell energy ATP, and by confirming an increasing ATP level in a muscle cell, it can be seen that the oxiracetam of the pharmaceutical composition for use according to the present invention has a protective effect against mitochondrial dysfunction and ATP deficiency due to skeletal muscle cell atrophy induced by TNF-α.

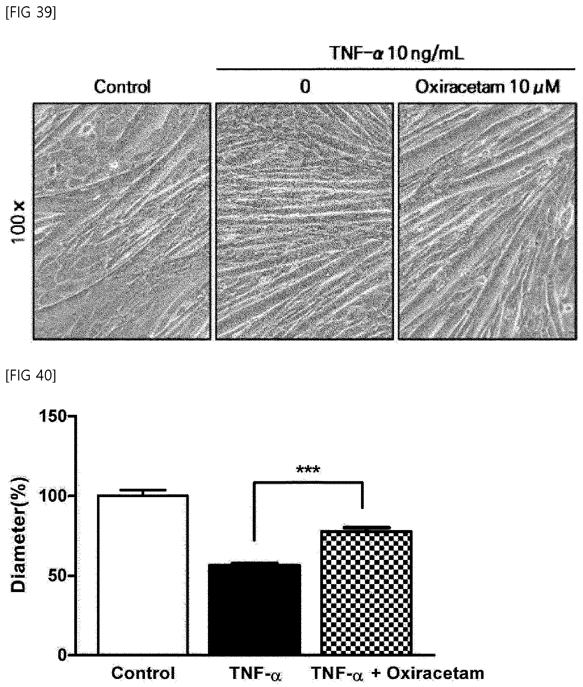

FIG. 39 is a set of images of TNF-α-treated myotube in the oxiracetam-treated group (Oxiracetam 10 µM) and the non-treated group (Oxiracetam 0 µM), captured by an optical microscope, and FIG. 40 is a graph showing the results of measuring the diameter of a TNF-α-treated myotube in the oxiracetam-treated group (Oxiracetam 10 µM) and the non-treated group (Oxiracetam 0 µM).

As shown in FIGS. 39 and 40, compared to the TNF-α-treated group (TNF-α), in the oxiracetam-treated group (Oxiracetam 10 µM), a significant increase in diameter of a myotube can be confirmed. Also, in the oxiracetam-treated group (Oxiracetam 10 µM), it can be seen that the diameter of a myotube recovers to a level similar to the control, but in the TNF-α-treated group (TNF-α), it is greatly reduced to half the level of the control.

Taken together, it was confirmed that atrophy of myotube is caused by TNF-α, and it can be seen that, through oxiracetam treatment, ATP production was increased by promoting mitochondrial activity, and the atrophy of myotube was suppressed to the control level.

### Experimental Example 8. Analysis of oxiracetam efficacy on cachexia

A mouse-derived C2C12 cell line was provided from the American Type Culture Collection (ATCC, CRL-1772; Manassas, VA, USA) and cultured at 37 °C in 5% CO₂. In the proliferation phase of the cells before differentiation, high concentration glucose Dulbecco's modified Eagle's medium (DMEM) containing 1% penicillin/streptomycin and 10% fetal bovine serum (FBS) medium were used, and in the differentiation induction, the FBS medium was replaced with a 2% horse serum (HS)-containing medium. The C2C12 myoblasts were seeded into a 96 well culture plate and a 6 well culture plate at 2×10⁵/well and 5×10³/well, respectively, and cultured at 37 °C in a CO₂ incubator. When the confluency reached approximately 90%, the medium was replaced with 2% horse serum (HS)-containing DMEM to induce differentiation of myoblasts. After 5 days, the C2C12 myotubes that had differentiated were treated with 500 nM anticancer agent (doxorubicin), which is an inflammatory factor, for 48 hours to cause myotube atrophy, thereby creating a cachexia cell model.

An oxiracetam-treated group (Oxiracetam) was prepared by treating oxiracetam at different concentrations (0, 0.01 µM, 0.1 µM, 1 µM, 10 µM and 30 µM) while treating an anticancer agent, and cells that were not treated during the same period were prepared as a control.

Two days after anticancer agent and oxiracetam treatment, the anticancer agent-treated group (Doxorubicin), the oxiracetam-treated groups (Oxiracetam 0.01 µM, 0.1 µM, 1 µM, 10 µM and 30 µM), and the control (control) were photographed using an optical microscope at a room temperature, and the diameter (Diameter, %) of a myotube in each group was measured and compared.

Statistical processing was performed using GraphPad Prism (GraphPad Software Inc., San Diego, CA, USA). The results of each repeated experiment were expressed as mean ± standard error of mean, and analyzed using the analysis of variance (Tukey's test), and statistical significance was acknowledged when the P-value was 0.05 or less.

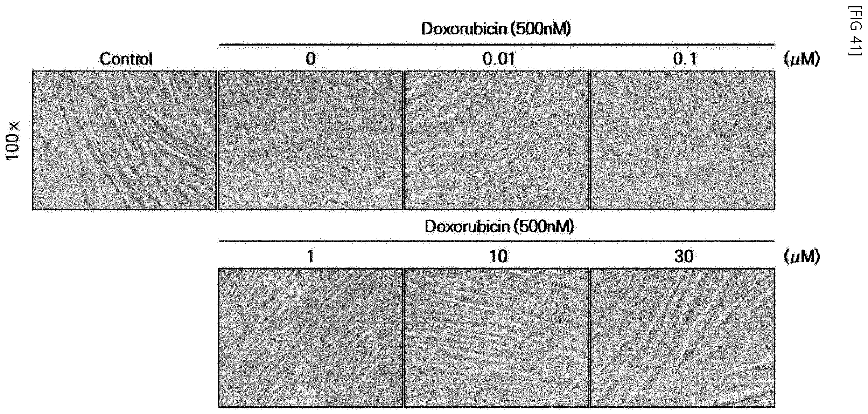

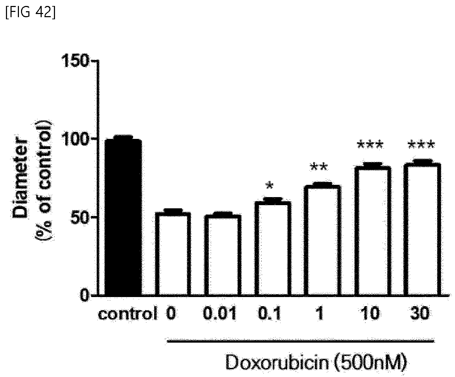

FIG. 41 is a set of images of the anticancer agent-treated group (doxorubicin), the oxiracetam-treated groups (oxiracetam 0.01 µM, 0.1 µM, 1 µM, 10 µM, 30 µM) and the control captured by an optical microscope, and FIG. 42 is a graph showing the results of measuring the diameter of myotube in the anticancer agent-treated group (doxorubicin), the oxiracetam-treated groups (oxiracetam 0.01 µM, 0.1 µM, 1 µM, 10 µM and 30 µM) and the control.

As shown in FIGS. 41 and 42, it can be confirmed that the anticancer agent-induced cachexia cell model (Doxorubicin) was significantly decreased in diameter of myotube compared to the control, resulting in atrophy. On the other hand, it can be seen that, in the oxiracetam-treated groups (Oxiracetam 0.01 µM, 0.1 µM, 1 µM, 10 µM and 30 µM), the diameter of myotube significantly recovered and greatly increased to the control (normal group) level.

Taken together, it was confirmed that cachexia causing atrophy of myotube cells is induced by the anticancer agent, and it can be seen that, through the oxiracetam treatment, the atrophy of myotube cells was suppressed to the control level. Therefore, it can be seen that the oxiracetam of the pharmaceutical composition for use according to the present invention also has an effect of preventing or treating cachexia as well as sarcopenia, muscular dystrophy.

Hereinafter, preparation examples for pharmaceuticals and food containing the oxiracetam of the pharmaceutical composition for use according to the present invention as an active ingredient will be described. By using the oxiracetam having an excellent effect in treatment or prevention of sarcopenia, muscular dystrophy or cachexia, pharmaceuticals and food compositions of Preparation Examples 1 and 2 were prepared by conventional methods according to the following compositions and composition ratios.

### [Preparation Example 1] Pharmaceuticals

### <1-1> Powder

A powder was prepared by mixing 50 mg of the oxiracetam and 2 g of crystalline cellulose and filling an airtight bag with the resulting mixture according to a conventional method of preparing a powder.

### <1-2> Tablet

A tablet was prepared by mixing 50 mg of the oxiracetam, 400 mg of crystalline cellulose, and 5 mg of magnesium stearate, and tableting the resulting mixture according to a conventional method of preparing a tablet.

### <1-3> Capsule

A capsule was prepared by mixing 30 mg of the oxiracetam, 100 mg of casein protein, 400 mg of crystalline cellulose, and 6 mg of magnesium stearate, and filling a gelatin capsule with the resulting mixture according to a conventional method of preparing a capsule.

### [Preparation Example 2] Food (not according to the invention)

### <2-1> Production of health food

A health food composition can be prepared by mixing 1000 mg of the oxiracetam, 70 µg of vitamin A acetate, 1.0 mg of vitamin E, 0.13 mg of vitamin B1, 0.15 mg of vitamin B2, 0.5 mg of vitamin B6, 0.2 µg of vitamin B12, 10 mg of vitamin C, 10 µg of biotin, 1.7 mg of nicotinamide, 50 µg of folic acid, 0.5 mg of calcium pantothenate, 1.75 mg of ferrous sulfate, 0.82 mg of zinc oxide, 25.3 mg of magnesium carbonate, 15 mg of monobasic potassium phosphate, 55 mg of dicalcium phosphate, 90 mg of potassium citrate, 100 mg of calcium carbonate, and 24.8 mg of magnesium chloride, and their mixing ratios may be arbitrarily modified. The above components may be mixed to prepare granules, and may be used in preparation of a health food composition.

### <2-2> Production of health drink (not according to the invention)

A health drink composition may be prepared by adding purified water to 100 mg of the oxiracetam, 100 g of oligosaccharide, 2 g of plum concentrate, and 1 g of taurine to make a total 900 mL mixture according to a method of preparing a health drink, heating the resulting solution at 85 °C for approximately 1 hour while stirring, filtering the resulting solution in a sterile 2 L container, and storing it in a refrigerator under sealed sterilization.

### <2-3> Chewing gum (not according to the invention)

A chewing gum was prepared in a conventional manner by mixing 20 wt% of gum base, 76.9 wt% of sucrose, 1 wt% of flavoring agent, 2 wt% of water, and 0.1 wt% of the oxiracetam.

### <2-4> Candy (not according to the invention)

A candy was prepared in a conventional manner by mixing 60 wt% of sucrose, 39.8 wt% of starch syrup, 0.1 wt% of a flavoring agent and 0.1 wt% of the oxiracetam.

### <2-5> Biscuit (not according to the invention)

A biscuit was prepared in a conventional manner by mixing 25.59 wt% of 1^{st} grade soft flour, 22.22 wt% of 1^{st} grade medium flour, 4.80 wt% of refined sugar, 0.73 wt% of table salt, 0.78 wt% of glucose, 11.78 wt% of palm shortening, 1.54 wt% of ammonium, 0.17 wt% of baking soda, 0.16 wt% of sodium bisulfite, 1.45 wt% of rice flour, 0.0001 wt% of vitamin B, 0.04 wt% of milk flavor, 20.6998 wt% of water, 1.16 wt% of skim milk powder, 0.29 wt% of substituted milk powder, 0.03 wt% of monobasic monohydrate, 0.29 wt% of salt spray, 7.27 wt% of oil spray, and 0.8301 wt% of the oxiracetam.

## Claims

1. A pharmaceutical composition for use in preventing or treating a muscle disease, comprising oxiracetam or a pharmaceutically acceptable salt or hydrate thereof as an active ingredient, and wherein the muscle disease is any one or more selected from the group consisting of atony, muscular atrophy, muscular dystrophy, muscular degeneration, myasthenia, myositis, cachexia, and senile sarcopenia.

2. The composition for use of claim 1, wherein the oxiracetam is represented by Formula 1 below.

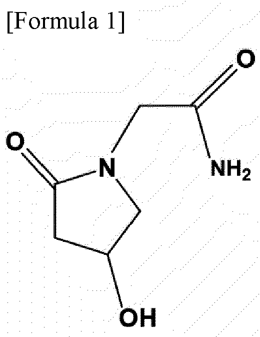

3. The composition for use of claim 1, wherein the muscular dystrophy is Duchenne muscular dystrophy (DMD).

4. The composition for use of claim 1, wherein the composition is administered by one or more selected from the group consisting of oral, intravenous, intramuscular, intraarterial, intramedullary, intrathecal, intracardiac, transdermal, subcutaneous, intraperitoneal, intranasal, enteral, local, sublingual and rectal administrations.

## Patentansprüche

1. Pharmazeutische Zusammensetzung zur Verwendung bei der Vorbeugung oder Behandlung einer Muskelerkrankung, umfassend Oxiracetam oder ein pharmazeutisch verträgliches Salz oder Hydrat davon als Wirkstoff, und wobei die Muskelerkrankung eine oder mehrere ausgewählt aus der Gruppe bestehend aus Atonie, Muskelatrophie, Muskeldystrophie, Muskeldegeneration, Myasthenie, Myositis, Kachexie und seniler Sarkopenie ist.

2. Die Zusammensetzung zur Verwendung nach Anspruch 1, wobei das Oxiracetam durch die nachstehende Formel 1 dargestellt wird.

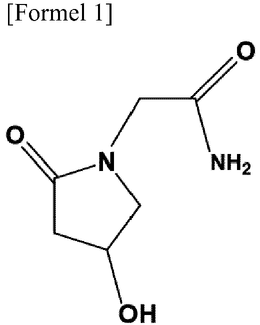

3. Die Zusammensetzung zur Verwendung nach Anspruch 1, wobei es sich bei der Muskeldystrophie um die Duchenne-Muskeldystrophie (DMD) handelt.

4. Die Zusammensetzung zur Verwendung nach Anspruch 1, wobei die Zusammensetzung durch eine oder mehrere verabreicht wird, die aus der Gruppe bestehend aus oraler, intravenöser, intramuskulärer, intraarterieller, intramedullärer, intrathekaler, intrakardialer, transdermaler, subkutaner, intraperitonealer, intranasaler, enteraler, lokaler, sublingualer und rektaler Verabreichung ausgewählt ist.

## Revendications

1. Composition pharmaceutique destinée à être utilisée pour prévenir ou traiter une maladie musculaire, comprenant de l'oxiracétam ou un sel ou un hydrate pharmaceutiquement acceptable de celui-ci en tant que principe actif, et dans laquelle la maladie musculaire est une ou plusieurs maladies choisies parmi le groupe constitué de l'atonie, de l'atrophie musculaire, de la dystrophie musculaire, de la dégénérescence musculaire, de la myasthénie, de la myosite, de la cachexie et de la sarcopénie sénile.

2. Composition d'utilisation selon la revendication 1, dans laquelle l'oxiracétam est représenté par la formule 1 ci-dessous.

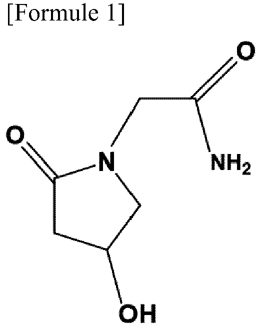

3. Composition d'utilisation selon la revendication 1, dans laquelle la dystrophie musculaire est la dystrophie musculaire de Duchenne (DMD).

4. Composition d'utilisation selon la revendication 1, dans laquelle la composition est administrée par une ou plusieurs voies choisies parmi le groupe comprenant les voies orale, intraveineuse, intramusculaire, intra-artérielle, intramédullaire, intrathécale, intracardiaque, transdermique, sous-cutanée, intrapéritonéale, intranasale, entérale, locale, sublinguale et rectale.
